# EUROPÄISCHE PATENTSCHRIFT

(11) **EP 1 252 327 B1**
(45) Veröffentlichungstag und Bekanntmachung des Hinweises auf die Patenterteilung: **25.08.2004**
(21) Anmeldenummer: 01902333.2
(22) Anmeldetag: 18.01.2001
(51) Int. Cl.: C12P 41/00, C12P 13/00, C12N 9/18

(54) **VERFAHREN ZUR ENZYMATISCHEN RACEMATSPALTUNG VON AMINOMETHYL-ARYL-CYCLOHEXANOL-DERIVATEN**
METHOD FOR THE ENZYMATIC RESOLUTION OF THE RACEMATES OF AMINOMETHYL-ARYL-CYCLOHEXANOL DERIVATIVES
PROCEDE POUR LA SEPARATION ENZYMATIQUE DE RACEMATES DE DERIVES D'AMINOMETHYL-ARYL-CYCLOHEXANOL

(30) Priorität: 04.02.2000 DE 10004926
(43) Veröffentlichungstag der Anmeldung: 30.10.2002
(73) Patentinhaber: Grünenthal GmbH, 52078 Aachen (DE)
(72) Erfinder: BUSCHMANN, Helmut, E-08950 Esplugues de Llobregat (ES); KAULARTZ, Dagmar, 52222 Stolberg (DE); GRIEBEL, Carsten, 52070 Aachen (DE); GAIS, Hans-Joachim, 52074 Aachen (DE)
(86) Internationale Anmeldenummer: PCT/EP2001/000522
(87) Internationale Veröffentlichungsnummer: WO 2001/057232

(56) Entgegenhaltungen:
- EP-A- 0 753 506
- WO-A-98/40053
- GAIS H -J ET AL: "Enzymatic resolution of analgesics: delta-hydroxytramadol, ?-hydroxytramadol and O-desmethyltramadol" TETRAHEDRON: ASYMMETRY,NL,ELSEVIER SCIENCE PUBLISHERS, AMSTERDAM, Bd. 11, Nr. 4, März 2000 (2000-03), Seiten 917-928, XP004192904 ISSN: 0957-4166
- E. FORRO AND F. FÜLÖP: "Enzymatic resolution of 2-dialkylaminomethylcyclopentanols and -cycloheptanols" TETRAHEDRON: ASYMMETRY,NL,ELSEVIER SCIENCE PUBLISHERS, AMSTERDAM, Bd. 10, Nr. 10, 21. Mai 1999 (1999-05-21), Seiten 1985-1993, XP002169071 ISSN: 0957-4166 in der Anmeldung erwähnt
- A. LUNA ET AL.: "Enzymatic resolution of (+/-)-cis and (+/-)-trans-1-aminoindan-2-ol and (+/-)-cis- and (+/-)-trans-2-aminoindan-1-ol" TETRAHEDRON: ASYMMETRY, Bd. 10, Nr. 10, 21. Mai 1999 (1999-05-21), Seiten 1969-1977, XP002169072 PERGAMON PRESS, ELSEVIER SCIENCE, NY, US in der Anmeldung erwähnt
- FORRO E ET AL: "Lipase-catalysed resolution of 2-dialkylaminomethylcyclohexanols" TETRAHEDRON: ASYMMETRY,NL,ELSEVIER SCIENCE PUBLISHERS, AMSTERDAM, Bd. 9, Nr. 3, 13. Februar 1998 (1998-02-13), Seiten 513-520, XP004131197 ISSN: 0957-4166 in der Anmeldung erwähnt
- FRANKUS E ET AL: "UEBER DIE ISOMERENTRENNUNG, STRUKTURAUFKLAERUNG UND PHARMAKOLOGISCHE CHARAKTERISIERUNG VON 1-(M-METHOXYPHENYL)-2-(DIMETHYLAMINOMETHYL )-CYC LOHEXAN-1-OL. ON SEPARARION OF ISOMERS, STRUCTURAL ELUCIDATION AND PHARMACOLOGICAL CHARACTERIZATION OF 1-(M-METHOXYPHENYL)-2-(DIMETHYLAM INOMETHYL)-CYCLOHEXAN-1-" ARZNEIMITTEL FORSCHUNG. DRUG RESEARCH,DE,EDITIO CANTOR. AULENDORF, Bd. 28, Nr. 1A, 1978, Seiten 114-121, XP000644506 ISSN: 0004-4172
- POULSEN L ET AL: "THE HYPOALGESIC EFFECT OF TRAMADOL IN RELATION TO CYP2D6" CLINICAL PHARMACOLOGY & THERAPEUTICS,US,MOSBY-YEAR BOOK, ST LOUIS, MO, Bd. 60, Nr. 6, 1. Dezember 1996 (1996-12-01), Seiten 636-644, XP000891863 ISSN: 0009-9236

## Beschreibung

Der Erfindung betrifft ein Verfahren zur enzymatischen Racematspaltung von Aminomethyl-Aryl-Cyclohexanol-Derivaten.

Die Behandlung chronischer und nicht chronischer Schmerzzustände hat in der Medizin eine große Bedeutung. Es besteht ein weltweiter Bedarf an gut wirksamen Schmerztherapien für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist. Dies zeigt sich in der großen Anzahl von wissenschaftlichen Arbeiten, die auf dem Gebiet der angewandten Analgetik bzw. der Grundlagenforschung zur Nociception in letzter Zeit erschienen sind.

Ein bekanntes Therapeutikum zur Behandlung starker Schmerzen ist Tramadolhydrochlorid -(1RS,2RS)-2-[(Dimethylamino)methyl]-1-(3-methoxyphenyl)cyclohexanol, Hydrochlorid. Aminomethyl-Aryl-Cyclohexanol-Derivate wie das Tramadol ((1RS, 2RS)-2-Dimethylaminomethyl-1-(3-methoxy-phenyl)-cyclo-hexanol, Hydrochlorid) können entsprechend eine analgetische Wirkung besitzen, aber auch hydroxylierte Tramadol-Derivate, wie sie z.B. in EP 753506 A1 beschrieben sind, oder sie können als Intermediate zur Herstellung von analgetisch wirksamen Substanzen verwendet werden (wie z.B. 4- oder 5-substituierte Tramadol-Analoga, die in der EP 753 506 A1 oder EP 780 369 A1 beschrieben sind). Gerade Tramadol nimmt unter den zentralwirksamen Analgetika insofern eine Sonderstellung ein, daß dieser Wirkstoff eine starke Schmerzhemmung ohne die für Opioide bekannten Nebenwirkungen hervorruft (J. Pharmacol. Exptl. Ther. 267, 331 (1993)), wobei sowohl die Enantiomeren von Tramadol als auch die Enantiomeren der Tramadolmetabolite an der analgetischen Wirkung beteiligt sind (J. Pharmacol. Exp. Ther. 260, 275 (1992)).

Wie man hieran erkennen kann, können Enantiomere deutlich unterschiedliche Wirkungen aufweisen, und es ist in vielfacher Hinsicht sehr wichtig, auch als Intermediate oder in Hinblick auf arzneirechtliche Zulassungen, Racemate enantiomerenrein auftrennen zu können.

Enzymatische Transformationen gehören inzwischen zu den Grundoperationen der präparativen organischen Chemie. Inzwischen sind auch zahlreiche industrielle Prozesse mit enzymatischen Schlüsselschritten etabliert, die heute weit über die enzymatische Racematspaltung von Aminosäuren hinausgehen. Eine aktuellere Übersicht über die Verwendung von Enzymen bei der Herstellung biologisch aktiver Verbindungen wird von Roberts und Williamson gegeben (S.M. Roberts, N.M. Williamson, *Current Organic Chemistry*, **1997,** Band *1*, 1-20).

Luana et al. (A. Luna, A. Maestro, C. Astorga, V. Gotor, *Tetrahedron: Asymmetry* **1999,** *10,* 1969-1977) beschreiben die enzymatische Racematspaltung via Umesterung von cyclischen α-Aminoalkoholen unter Verwendung von Lipasen und Vinylacetat als Acyldonor. Diese Publikation ist deshalb von Bedeutung, da gezeigt wird, dass Substrate mit Aminoalkohol-Funktionalität verwendet werden können. 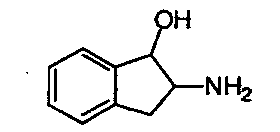 Forró und Fülöp (E. Forró, F. Fülöp, *Tetrahedron: Asymmetry* **1999,** *10,* 1985-1993, E. Forró, L. Kanerva, F. Fülöp, *Tetrahedron: Asymmetry* **1998,** *9,* 513-520) beschreiben die enzymatische Racematspaltung von reduzierten cyclischen Mannichbasen des folgenden Typs:

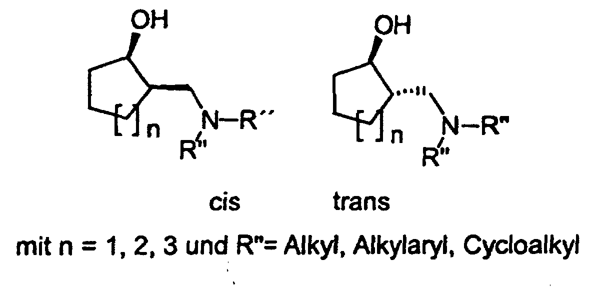

Die Autoren stellen den Bezug zum Tramadol in der Einleitung her und verweisen im Einleitungstext auf die Verwendung dieser Verbindungen als Bausteine für potentiell analgetisch wirksame Substanzen.

Bei der Entwicklung enzymatischer Verfahren ist neben dem geeigneten Enzymsystem das Auffinden der geeigneten Reaktionsparameter für das Gelingen des Verfahrens entscheidend.

Die Herstellung der enantiomerenreinen Aminomethyl-Aryl-Hexanol-Derivate, insbesondere der 4- oder 5-hydroxylierten Tramadol-Derivate, ist bisher über eine fraktionierte Kristallisation diastereomerer Salze wie z.B. Tartrate, Dibenzoyltartrate oder Dobenzoyltartrate nicht gelungen. Präparative chromatographische Verfahren sind für die Bereitstellung enantiomerenreiner Verbindungen im Multigramm-Massstab nur in bestimmten Fällen einsetzbar. Geeignete chromatographische Bedingungen für die präparative Trennung wurden bisher ebenfalls nicht aufgefunden.

Aufgabe der vorliegenden Erfindung war es daher, geeignete Verfahren für die enantiomerenreine Trennung der Enantiomere von Aminomethyl-Aryl-Hexanol-Derivaten, insbesondere der 4- oder 5-hydroxylierten Tramadol-Derivate, - auch im größeren Maßstab - aufzufinden.

Gegenstand der Erfindung sind daher Verfahren zur enzymatischen Racematspaltung von Aminomethyl-Aryl-Cyclohexanol-Derivaten der allgemeinen Formel I 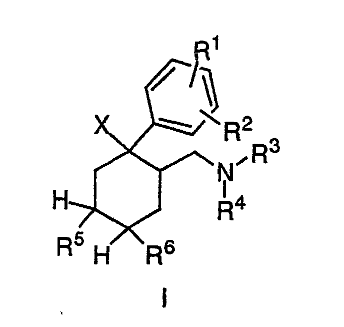 , worin X ausgewählt ist aus
H, F, Cl, Br, I, CF₃, O-S(O₂)-C₆H₄-pCH₃, OR¹⁴ oder OC(O)R¹⁴, wobei R¹⁴ ausgewählt ist aus
H; C₁-C₁₀-Alkyl, C₂-C₁₀-Alkenyl oder C₂-C₁₀-Alkinyl, jeweils verzweigt oder unverzweigt, einfach oder mehrfach substituiert oder unsubstituiert; C₃-C₇-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl oder Alkylheteroaryl, gesättigt oder ungesättigt, einfach oder mehrfach substituiert oder unsubstituiert; Aryl oder Heteroaryl, jeweils einfach oder mehrfach substituiert oder unsubstituiert;
R³, R⁴ unabhängig voneinander ausgewählt sind aus
H, C₁-C₁₀-Alkyl, C₂-C₁₀-Alkenyl oder C₂-C₁₀-Alkinyl, jeweils verzweigt oder unverzweigt, einfach oder mehrfach substituiert oder unsubstituiert; C₃-C₇-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl oder Alkylhereroaryl, gesättigt oder ungesättigt, einfach oder mehrfach substituiert oder unsubstituiert; Aryl oder Heteroaryl, jeweils einfach oder mehrfach substituiert oder unsubstituiert;
oder
R³ und R⁴ zusammen ein C₃-C₇-Cycloalkyl, gesättigt oder ungesättigt, einfach oder mehrfach substituiert oder unsubstituiert,bilden, bzw. einen entsprechenden Heterocyclus, bei dem ein C-Atom im Ring durch S, O oder NR¹⁵ ersetzt ist, mit R¹⁵ ausgewählt aus
H, C₁-C₁₀-Alkyl, C₂-C₁₀-Alkenyl oder C₂-C₁₀-Alkinyl, jeweils verzweigt oder unverzweigt, einfach oder mehrfach substituiert oder unsubstituiert;
R¹ und R² unabhängig voneinander wie weiter unten erläutert R¹⁰ oder YR¹⁰ sind
und
jeweils einer von den Substituenten R⁵ und R⁶ H und der andere OH entspricht, dadurch gekennzeichnet, daß je nach gewünschtem Enantiomer der Aminomethyl-Aryl-Cyclohexanol-Derivate der allgemeinen Formel I
**entweder** in der Reaktionsalternative I
das Racemat von Verbindungen gemäß Formel I zunächst verestert und anschließend enzymatisch transformiert wird und die entstehenden enantiomerenreinen Verbindungen getrennt werden
**oder** in der Reaktionsaltemative II
das Racemat von Verbindungen gemäß Formel I in Gegenwart eines Esters enzymatisch transformiert wird und die entstehenden enantiomerenreinen Verbindungen getrennt werden.

Hierbei wird insbesondere ausgenutzt, daß die Reaktionsaltemativen I und 11 als komplementäre Verfahren anzusehen sind, da bei der enzymatischen Transformation des racemischen Gemisches die jeweils entgegengesetzte Stereochemie induziert wird.

Bei der Reaktionsaltemative I transformiert man enzymatisch eine racemische Verbindung gemäß Formel II 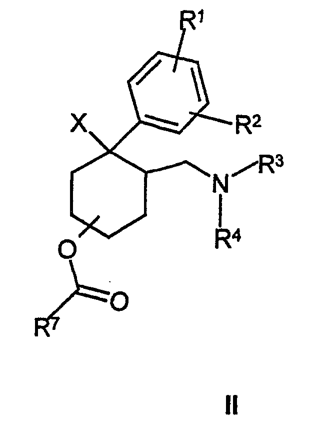 , in der der Substituent OC(O)R⁷ der Position von R⁵ oder R⁶ in Formel I entspricht und R⁷ ausgewählt ist aus C₁-C₆-Alkyl, unsubstituiert oder einfach oder mehrfach substituiert; als freie Base oder in Form ihres Salzes in einem Lösungsmittel mit einer Lipase oder Esterase und trennt die entstehende enantiomerenreinen Verbindungen gemäß Formeln III und Ia 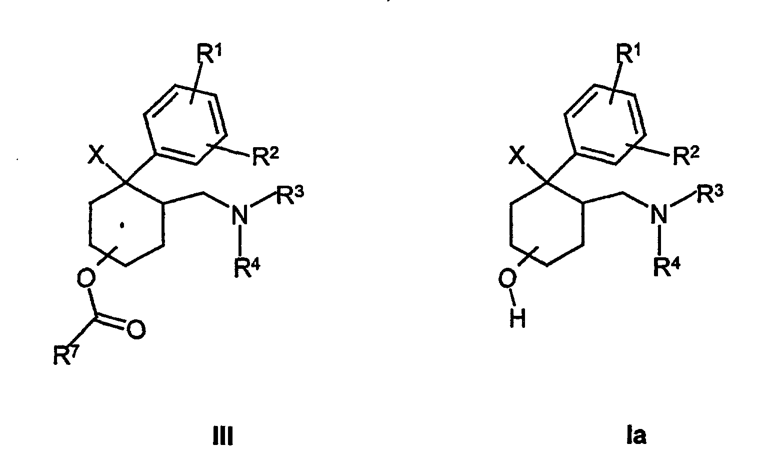 , wobei Verbindungen nach Formel la Verbindungen nach Formel I entsprechen und der Substituent OH der Position von R⁵ oder R⁶ in Formel I entspricht.

Besonders bevorzugt ist es bei der Reaktionsalternative I, wenn R⁷ in Formeln II und III Chloracetyl-, Butyl- oder Pentyl- ist.

Als Enzym wird in der Reaktionsalternative I vorzugsweise eine Esterase, insbesondere eine Schweineleberesterase, verwendet.

Das bevorzugte Lösungsmittel bei der Reaktionsaltemative I ist ein wässriges Puffersystem, das vorzugsweise einen pH zwischen 6,0 und 8,0 - vorzugsweise einen pH zwischen 7,0 und 7,5, aufweist. Dabei ist es auch günstig, wenn das Lösungsmittel ein wässriges Puffersystem mit einem für das verwendete Enzym physiologischen pH ist. Dabei ist es besonders günstig, wenn dem wäßrigen Puffersystem ein oder mehrere organische/s Lösungsmittel, vorzugsweise Aceton oder Butanol, bis zu einem Volumenprozentanteil zwischen 1 und 50%, vorzugsweise 5 und 20 %, insbesondere 20% zugefügt ist/sind.

Weiter ist es bevorzugt, insbesondere bei wässrigen Puffersystem, in der Reaktionsalternative I die Verbindung gemäß Formel II als Hydrochtoridsalz einzusetzen.

Dabei ist es von besonderer Bedeutung, daß insbesondere bei der enzymatischen Hydrolyse des Buttersäureesters des 4-Hydroxytramadols aber auch in anderen Fällen gemäß Reaktionsalternative I - gerade mit wässrigem Puffersystem - der Einsatz des Salzes, insbesondere Hydrochlorids und nicht der Base, zu besseren Ergebnissen führen kann. Die Base ist im wässrigen Puffersystem häufig nicht in ausreichender Menge löslich. Weiter ist es besonders bemerkenswert, daß bei Aceton- und Butanolzusatz eine deutliche Verbesserung des Verfahrens, insbesondere auch bei Einsatz des Hydrochlorides, zu beobachten ist. Das gilt besonders für die Reaktionsgeschwindigkeit. Insbesondere ein Aceton- oder Butanolzusatz zum wässrigen Puffer bis zu zwischen 5 und 20%, vorzugsweise 20%, der GesamtVolumens ist bezüglich Selektivität und Reaktionsgeschwindigkeit häufig optimal.

Auch im Stand der Technik ist bei enzymatischen Trennungen der Einsatz von Aminohydrochloriden bisher noch nicht beschrieben worden.

Zur Herstellung des Esters, der Verbindungen gemäß Formel II, in Reaktionsalternative I werden racemische Verbindungen gemäß Formel I 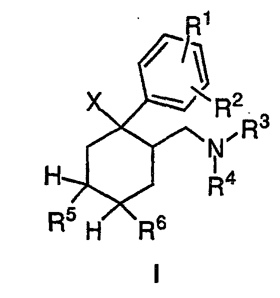 mit Basen, vorzugsweise Kalium-tert-butylat oder Natriumhydrid, in einem Lösungsmittel, vorzugsweise Tetrahydrofuran oder Dimethylformamid, in die Alkoholate überführt und anschließend unter Zugabe der entsprechenden Säurehalogenide in die racemischen Ester gemäß Formel II 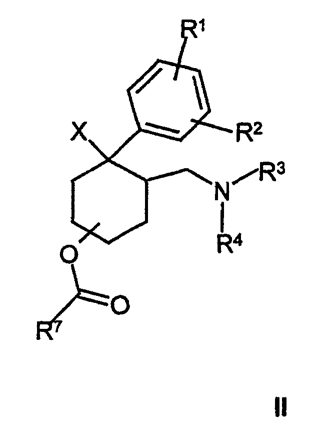 , in denen der Substituent OC(O)R⁷ der Position von R⁵ oder R⁶ in Formel I entspricht, umgesetzt. So können vorzugsweise die Ester gemäß Formel II hergestellt werden.

Bei der Reaktionsalternative II transformiert man enzymatisch eine racemische Verbindung gemäß Formel I 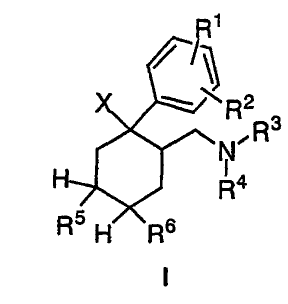 als freie Base oder in Form ihres Salzes in einem Lösungsmittel mit einem Ester gemäß Formel IV 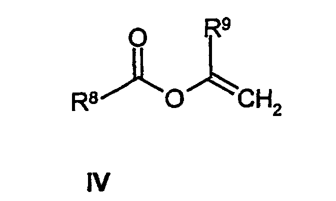 , worin unabhängig voneinander R⁸ C₁-C₆-Alkyl, substituiert oder unsubstituiert; und R⁹ H oder C₁-C₆-Alkyl, substituiert oder unsubstituiert bedeuten, einsetzt, mit einer Lipase oder Esterase und trennt die entstehenden enantiomerenreinen Verbindungen gemäß Formeln V und Ib. 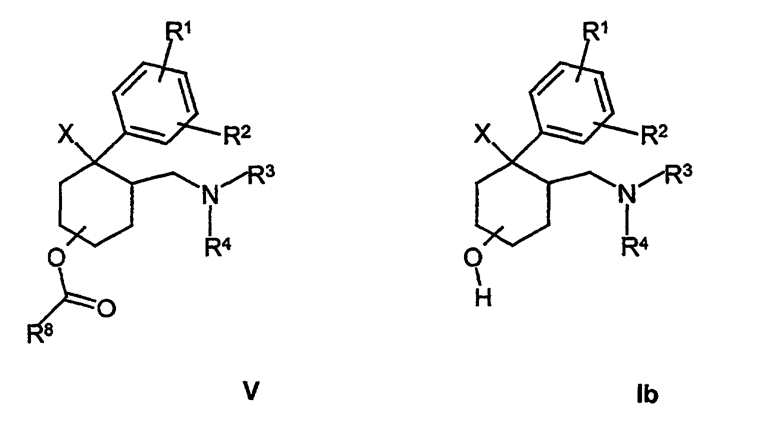 , wobei Verbindungen nach Formel Ib Verbindungen nach Formel I entsprechen und der Substituent OH der Position von R⁵ oder R⁶ in Formel I entspricht.

Dabei ist es besonders bevorzugt, wenn bei der Reaktionsaltemative II in den Estern gemäß Formeln IV und V R⁸ Methyl oder Ethyl und/oder R⁹ gemäß Formel IV H oder Methyl bedeuten.

Insbesondere der Ester gemäß Formel IV ist vorzugsweise Vinylpropionat, Vinylacetat oder Isopropenylacetat.

Als Enzym wird bei der Reaktionsalternative II vorzugsweise eine Lipase, insbesondere eine Lipase aus *candida rugosa, Candida cylindracea* oder *Pseudomonas cepacia* verwendet.

Als weiter besonders günstig hat es sich erwiesen, als Lösungsmittel bei der Reaktionsalternative II ein organisches Lösungsmittel, vorzugsweise Toluol, zu verwenden.

Ein entscheidender Vorteil des erfindungsgemäßen Verfahrens nach beiden Reaktionsalternativen ist die leicht zu erreichende Trennung der enantiomerenreinen Verbindungen nach Abschluß der enzymatischen Transformation. Dabei werden die Ester/Alkoholgemische nach Abschluß der enzymatischen Transformation durch pH-selektive Extraktion getrennt. Es kann vorteilhafterweise auf eine chromatographische Trennung verzichtet werden. Durch Einstellen des geeigneten pH-Wertes lassen sich Ester und Alkohol aufgrund hinreichend unterschiedlicher log P-Werte extraktiv voneinander trennen, insbesondere durch durch pH-selektive Extraktion. Damit ist ein Upscaling problemlos möglich und besonders einfach technisch durchzuführen.

Die gefundenen enzymatischen Verfahren nach beiden Reaktionsaltemativen stellen die zur Zeit einzige Möglichkeit dar, Aminomethyl-Aryl-Cyclohexanol-Derivate, insbesondere hydroxylierte Tramadol-Derivate, im Multigramm-Massstab mit der ausreichenden Enantiomerenreinheit herzustellen.

Insgesamt, aber insbesondere bei der Esterspaltung nach Reaktionsalternative I, kann der Umsatz bis fast 50 % geführt werden, ohne dass die Selektivität wie bei vielen vergleichbaren enzymatischen Racematspaltungen drastisch erniedrigt wird. Eine Überverseifung war unter den angewandten Reaktionsbedingungen nicht zu beobachten.

Erfindungsgemäß sind die Substituenten R¹ und R² in den Formeln I, Ia, Ib, II, III und V unabhängig voneinander ausgewählt aus R¹⁰ oder YR¹⁰ mit Y = C₁-C₁₀-Alkyl, C₂-C₁₀-Alkenyl oder C₂-C₁₀-Alkinyl, verzweigt oder unverzweigt, einfach oder mehrfach substituiert oder unsubstituiert, wobei R¹⁰ ausgewählt ist aus
H, F, Cl, Br, I, CN, NO₂, C₁-C₈-Alkyl, C₂-C₈-Alkenyl oder C₂-C₈-Alkinyl, jeweils verzweigt oder unverzweigt, einfach oder mehrfach substituiert oder unsubstituiert; C₃-C₇-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder N ersetzt ist; Aryl oder Heteroaryl, jeweils einfach oder mehrfach substituiert oder unsubstituiert;
OR¹¹, OC(O)R¹¹, OC(O)OR¹¹, OC(S)R¹¹, C(O)R¹¹, C(O)OR¹¹, C(S)R¹¹, C(S)OR¹¹, SR¹¹, S(O)R¹¹ bzw. S(O₂)R¹¹, wobei R¹¹ ausgewählt ist aus
H, C₁-C₁₈-Alkyl, C₂-C₁₈-Alkenyl oder C₂-C₁₈-Alkinyl, jeweils verzweigt oder unverzweigt, einfach oder mehrfach substituiert oder unsubstituiert; C₃-C₇-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder N ersetzt ist; Alkylaryl oder Alkylheteroaryl, gesättigt oder ungesättigt, einfach oder mehrfach substituiert oder
NR¹²R¹³, C(O)NR¹²R¹³ oder S(O₂)NR¹²R¹³, wobei R¹² und R¹³ unabhängig voneinander ausgewählt sind aus
H, C₁-C₁₈-Alkyl, C₂-C₁₈-Alkenyl oder C₂-C₁₈-Alkinyl, jeweils verzweigt oder unverzweigt, einfach oder mehrfach substituiert oder unsubstituiert; C₃-C₇-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder N ersetzt ist; Alkylaryl oder Alkylheteroaryl, gesättigt oder ungesättigt, einfach oder mehrfach substituiert oder unsubstituiert; Aryl oder Heteroaryl, jeweils einfach oder mehrfach substituiert oder unsubstituiert;
oder
R¹² und R¹³ zusammen ein C₃-C₇-Cycloalkyl, gesättigt oder ungesättigt, einfach oder mehrfach substituiert oder unsubstituiert, bilden, bzw. einen entsprechenden Heterocyclus, bei dem ein C-Atom im Ring durch S, O oder N ersetzt ist;
oder
R¹ und R² zusammen -CH=CH-CH=CH- bilden, wobei das entstehende Naphthylsystem ein- oder mehrfach substituiert sein kann.

Die folgende Definitionen gelten für die vollständige Beschreibung der gesamten hier dargestellten Erfindung, insbesondere auch weiter vorangestellte Abschnitte und Definitionen von Resten, soweit nicht ausdrücklich etwas anderes definiert wurde.

Dabei versteht man im Zusammenhang mit Alkyl, Alkenyl, Alkinyl und Cycloalkyl bzw. dem "entsprechenden Heterocyclus" unter dem Begriff substituiert im Sinne dieser Erfindung die Substitution eines Wasserstoffrestes durch F, Cl, Br, I, NH₂, SH oder OH,
wobei unter mehrfach substituierten Resten Reste zu verstehen sind, die sowohl an verschiedenen als auch an gleichen Atomen mehrfach substituiert sind, beispielsweise dreifach am gleichen C-Atom wie im Falle von CF₃ oder an verschiedenen Stellen wie im Falle von -CH(OH)-CH=CH-CHCl₂.
Weiter bedeutet -C(O)-
, was auch für -C(S)- oder 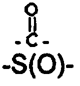 bzw. -S(O₂)- gilt.

Der Ausdruck "C₁-C₈-Alkyl" bzw. "C₁-C₁₀-Alkyl" bedeutet im Sinne dieser Erfindung Kohlenwasserstoffe mit 1 bis 8 bzw. 10 Kohlenstoffatomen. Beispielhaft seien Methyl, Ethyl, Propyl, Isopropyl, n-Butan, sek-Butyl, tert-Butyl, n-Pentan, Neopentyl, n-Hexan, n-Heptan, n-Octan, n-Nonan oder n-Decan genannt

Der Ausdruck "C₁-C₁₈-Alkyl" bedeutet im Sinne dieser Erfindung Kohlenwasserstoffe mit 1 bis 18 Kohlenstoffatomen. Beispielhaft seien Methyl, Ethyl, Propyl, Isopropyl, n-Butan, sek-Butyl, tert-Butyl, n-Pentan, Neopentyl, n-Butan, sek-Butyl, tert-Butyl, n-Hexan, n-Heptan, n-Octan, n-Nonan, n-Decan, n-Undecan, n-Dodecan, n-Tridecan, n-Tetradecan, n-Pentadecan, n-Hexadecan, n-Heptadecan oder n-Octadecan unsubstituiert oder ein oder mehrfach substituiert genannt.

Der Ausdruck "C₂-C₁₀-Alkenyl" bzw. "C₂-C₁₀-Alkinyl" oder "C₂-C₁₈-Alkenyl" bzw. "C₂-C₁₈-Alkinyl" bedeutet im Sinne dieser Erfindung Kohlenwasserstoffe mit 2 bis 10 bzw. 2 bis 18 Kohlenstoffatomen. Beispielhaft genannt seien Propenyl, Butenyl, Pentenyl, Hexenyl, Heptenyl, Octenyl unsubstituiert oder ein oder mehrfach substituiert bzw. Propinyl, Butinyl, Pentinyl, Hexinyl, Heptinyl, Octinyl unsubstituiert oder ein oder mehrfach substituiert.

Der Ausdruck C₃-C₇-Cycloalkyl bedeutet im Sinne dieser Erfindung cyclische Kohlenwasserstoffe mit 3 bis 7 Kohlenstoffatomen. Beispielhaft seien Cyclopropyl, Cyclobutyl, Cyclopentyl, Cyclohexyl, Cycloheptyl, Cyclopentenyl, Cyclohexenyl oder Cycloheptenyl gesättigt oder ungesättigt, unsubstituiert oder ein oder mehrfach substituiert genannt. Dabei versteht man im Sinne der Erfindung unter einem "entsprechenden Heterocyclus" ein C₃-C₇-Cycloalkyl, bei dem mindestens ein C-Atom im Ring durch S, O oder N ersetzt ist. Beispielhaft seien hierfür Pyrrolidin, Pyran, Thiolan, Piperidin oder Tetrahydrofuran aufgeführt.

Der Ausdruck "Aryl" bedeutet im Sinne dieser Erfindung Phenyle, Naphthyle oder Anthracenyle. Die Aryl-Reste können auch mit weiteren Ringen kondensiert sein.

Der Ausdruck "Heteroaryl" bedeutet im Sinne dieser Erfindung gegebenenfalls mit einem ankondensierten Ringsystem versehene, aromatische Verbindungen, die wenigstens ein Heteroatom aus der Gruppe Stickstoff, Sauerstoff und/oder Schwefel enthalten. In dieser Gruppe seien beispielhaft Thiophen, Furan, Pyrrol, Pyridin, Pyrimidin, Chinolin, Isochinolin, Phtlalazin oder Chinazolin aufgeführt.

Der Ausdruck "Alkylaryl" bzw. "Alkylheteroaryl" bedeutet im Sinne dieser Erfindung mindestens mit C₁-C₆-Alkylen substituierte Aryle bzw. Heteroaryle, wobei die Ausdrücke Aryl, Heteroaryl und Alkyl die gleiche Bedeutung haben wie oben, in denen die Bindung über den Alkylrest erfolgt.
In Bezug auf "Aryl", "Alkylaryl", "Heteroaryl" oder "Alkylheteroaryl" versteht man im Sinne dieser Erfindung unter ein- oder mehrfach substituiert die Substitution des Ringsystems mit F, Cl, Br, I, NH₂, SH, OH, CF₃; =O oder =S; ein- oder mehrfach substituiertem oder unsubstituiertem C₁-C₆-Alkyl, C₁-C₆-Alkoxy, C₂-C₈-Alkenyl, C₂-C₈-Alkinyl; Phenyl oder Benzyl; an einem oder verschiedenen Atomen.
Insbesondere vorteilhaft ist es, wenn R¹ in den Formeln I, la, Ib, II, III und V gleich R¹⁰ ist, wobei R¹⁰ ausgewählt ist aus
H, F, Cl, Br, I, CF₃, NO₂, NH₂; C₁-C₄-Alkyl oder C₂-C₄-Alkenyl, verzweigt oder unverzweigt, einfach oder mehrfach substituiert oder unsubstituiert; OR¹¹, C(O)OR¹¹ bzw. SR¹¹ wobei R¹¹ ausgewählt ist aus
H; C₁-C₄-Alkyl, verzweigt oder unverzweigt, einfach oder mehrfach substituiert oder unsubstituiert; vorzugsweise H, CF₃ oder CH₃,
oder S(O₂)NR¹²R¹³, wobei R¹² und R¹³ unabhängig voneinander ausgewählt sind aus
H; C₁-C₄-Alkyl, verzweigt oder unverzweigt, einfach oder mehrfach substituiert oder unsubstituiert;
, wobei insbesondere bevorzugt ist, daß R¹ ausgewählt ist aus
H, F, Cl, OH, CH₃, C₂H₅, C₂H₃, CF₃, SCH₃, OCF₃, OCH₃, OC₂H₅, C(O)OCH₃, C(O)OC₂H₅, vorzugsweise m-OCH₃.

Insbesondere kann der Substituent R² in den Formeln I, la, Ib, II, III und V gleich R¹⁰ sein, wobei R¹⁰ ausgewählt ist aus
H, F, Cl, Br, I, SCH₃; C₁-C₄-Alkyl, C₂-C₄-Alkenyl verzweigt oder unverzweigt, einfach oder mehrfach substituiert oder unsubstituiert, vorzugsweise CF₃; OR¹¹, mit R¹¹ ausgewählt aus C₁-C₄-Alkyl, verzweigt oder unverzweigt, einfach oder mehrfach substituiert oder unsubstituiert, vorzugsweise CH₃;
, wobei insbesondere bevorzugt ist, daß R² = H.

Es ist weiter besonders bevorzugt, wenn X in den Formeln I, Ia, Ib, II, III und V ausgewählt ist aus
H, F, Cl, OH, CF₃, O-S(O₂)-C₆H₄-pCH₃ bzw. OC(O)R¹² mit R¹² = H; C₁-C₄-Alkyl oder C₂-C₄-Alkenyl, verzweigt oder unverzweigt, ein- oder mehrfach substituiert oder unsubstituiert,
vorzugsweise H, F, Cl, OH, O-S(O₂)-C₆H₄-pCH₃, OC(O)R¹² mit
R¹² = C₁-C₄-Alkyl, vorzugsweise CH₃;
, wobei insbesondere bevorzugt ist, daß X gleich OH, F oder Cl, vorzugsweise OH ist.

Es ist weiter ein bevorzugter Gegenstand der Erfindung , wenn R³ und R⁴ in den Formel I, II, III und V unabhängig voneinander ausgewählt sind aus
C₁-C₄-Alkyl, verzweigt oder unverzweigt, einfach oder mehrfach substituiert oder unsubstituiert, vorzugsweise CH₃, oder
R³ und R⁴ zusammen ein C₃-C₇-Cycloalkyl, gesättigt oder ungesättigt, einfach oder mehrfach substituiert oder unsubstituiert, bilden,
wobei insbesondere bevorzugt ist, daß R³ und R⁴ jeweils CH₃ bedeuten.

Ein weiterer Gegenstand der Erfindung sind auch die Zwischenprodukte nach Formel II. Die Definition der aufgeführten Reste R¹-R⁴ und X sowie R⁷ ist bereits weiter oben beschrieben worden wie auch ein bevorzugtes Herstellungsverfahren für Produkte nach Formel II im Rahmen der Reaktionsaiternative I. Die Verbindungen gemäß Formel 11 sind sehr geeignete Analgetika und sind auch bei weiteren Indikationen einsetzbar. Sie sind daher in Form ihrer Diastereomere oder Enantiomere sowie ihrer freien Base oder eines mit einer physiologisch verträglichen Säure gebildeten Salzes, insbesondere des Hydrochloridsalzes, zur Herstellung eines Arzneimittels zur Behandlung von Schmerz, insbesondere Migräne, Akutschmerz sowie neuropathischem oder chronischem Schmerz, von inflammatorischen und allergischen Reaktionen, Depressionen, Drogenund/oder Alkoholmißbrauch, Gastritis, cardiovaskulären Erkrankungen, Atemwegserkrankungen, Husten, seelischen Erkrankungen und/oder Epilepsie sowie insbesondere von Harninkontinenz, Juckreiz und/oder Diarrhoe geeignet.

Im folgenden wird die Erfindung weiter durch Beispiele erläutert, ohne sie darauf zu beschränken.

### Beispiele

Die folgenden Beispiele zeigen erfindungsgemäße Verfahren.

Dabei gelten generell folgende Angaben:

Die eingesetzten Chemikalien und Lösungsmittel wurden kommerziell bei den herkömmlichen Anbietern erworben (Acros, Avocado, Aldrich, Fluka, Lancaster, Maybridge, Merck, Sigma, TCI etc. oder synthetisiert).

### Beispiel 1

### Darstellung der Carbonsäureester von Hydroxy-Tramadolen

### (1 SR, 3 RS, 4 RS)-Buttersäure- 3-dimethylaminomethyl-4-hydroxy-4-(3-methoxyphenyl)-cyclohexyl ester, Hydrochlorid (rac-1)

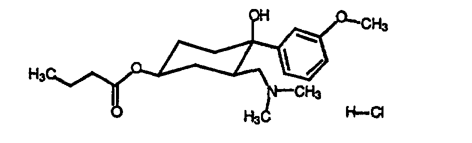

250 g ( 0.89 mol) (1 RS, 2 RS, 4 SR)-2-Dimethylaminomethyl-1-(3-methoxy-phenyl)-cyclohexan-1,4-diol **rac-2** wurden in 2500 ml getrocknetem Tetrahydrofuran suspendiert und portionsweise mit 226 g Kalium-*tert*.-butylat (2.01 mol) unter Eisbadkühlung versetzt, so dass die Innentemperatur nicht 30 °C überschritt. Nach beendeter Zugabe wurde noch eine Stunde bei Raumtemperatur nachgerührt. Anschließend wurden unter Eisbadkühlung 127 ml (130,3 g, 1,22 mol) Buttersäurechlorid zugegeben, wobei die Innentemperatur zwischen 5 und 10 °C lag. Nach vollständiger Zugabe wurde noch 15 Szunden bei Raumtemperatur nachgerührt. Zur Hydrolyse wurden unter erneuter Eisbadkühlung 1187 ml einer 1-molaren wässrigen Natriumhydrogencarbonatlösung zugetropft. Nach Phasentrennung wurde die wässrige Phase noch zweimal mit 500 ml Essigsäureethylester extrahiert. Die vereinigten organischen Phasen wurden über Natriumsulfat getrocknet. Nach der destillativen Entfernung des Lösungsmittels wurde der Rückstand (277.4 g) in das Hydrochlorid überführt. Hierzu wurden die 277.4 g Rohprodukt in ein Lösungsmittelgemisch bestehend aus 270 ml Ethanol und 1350 ml Aceton gelöst. Nach Zugabe von einem Motequivalent Trimethylchlorsilan und einem Molequivatent Wasser kristallisierte das Hydrochlorid aus. Nach 15 stündigem Stehenlassen bei 15 °C wurde abgesaugt und nach Trocknung konnten 273.2 g Hydrochlorid in 89 %iger Ausbeute erhalten werden.

### Beispiel 2

### Darstellung der Carbonsäureester der Hydroxy-tramadole

### (1 SR, 3 RS, 4 RS, Buttersäure- 4-dimethylaminomethyl-3-hydroxy-3-(3-methoxyphenyl)-cyclohexyl ester, Hydrochlorid (rac-3)

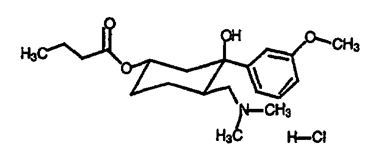

Analog zur Darstellung des (1 SR, 3 RS, 4 RS)-Buttersäure- 3-dimethylaminomethyl-4-hydroxy-4-(3-methoxy-phenyl)-cyclohexyl ester, Hydrochlorids **rac-1** konnte aus (1 RS, 3 SR, 6 RS)-6-Dimethylaminomethyl-1-(3-methoxy-phenyl)-cyclohexan-1,3-diol **rac-4** der Ester **rac-3** in 85 %iger Ausbeute erhalten werden.

### Beispiel 3:

### Enzymatische Esterhydrolyse

### Schweineleberesterase-katalysierte Hydrolyse des (1 SR, 3 RS, 4 RS)-Buttersäure- 3-dimethylaminomethyl-4-hydroxy-4-(3-methoxy-phenyl)-cyclohexyl ester, Hydrochlorids (rac-1)

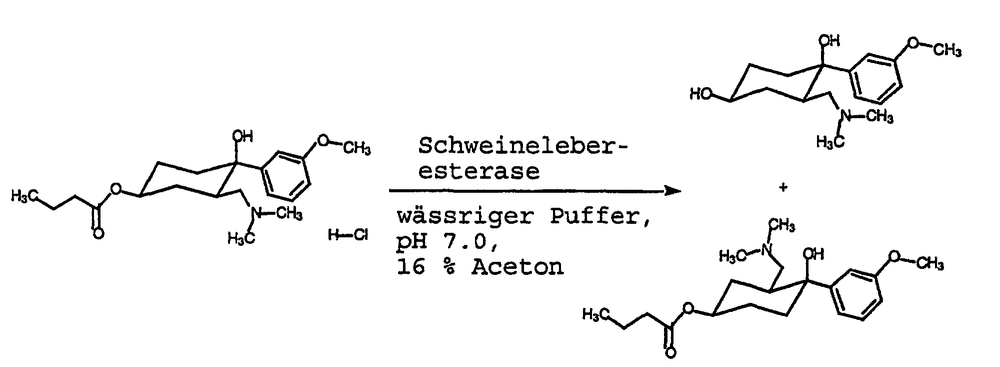

(-)-(1 R, 3 S, 4 S)-Buttersäure- 3-dimethylaminomethyl-4-hydroxy-4-(3-methoxy-phenyl)-cyclohexyl ester (**(-)-1**)
und
(+)-(1 R, 2 R, 4 S)-2-Dimethylaminomethyl-1-(3-methoxy-phenyl)-cyclohexan-1,4-diol (**(+)-2**)

72 g (0.19 mol) des (1 SR, 3 RS, 4 RS)-Buttersäure- 3-dimethylaminomethyl-4-hydroxy-4-(3-methoxy-phenyl)-cyclohexyl ester, Hydrochlorid **rac-1** wurden in 620 ml wässriger Phosphatpufferlösung pH 7 (Fa. Merck, Art.-Nr. 1.09439.100) gelöst und mit 140 ml Aceton versetzt. Nach 10 minütigem Rühren entstand eine klare Lösung. Anschliessend wurden 0.62 g Schweineleberesterase (Chirazyme E1 der Fa. Roche Diagnostics, Lyophilisat, 40 Units/mg) und 150 ml einer 1-molaren wässrigen Natriumhydrogencarbonatlösung in einer Portion zugegeben, so dass sich ein pH-Wert von 7.5 einstellte. Das Reaktionsgemisch wurde 21 Stunden bei Raumtemperatur gerührt. Zur Beendigung der reaktion wurde das Puffersystem jeweils zweimal mit 450 ml Diisopropylether und jeweils zweimal mit einem Lösungsmittelgemisch aus Diisopropylether und Diethylether im Verhältnis 1 : 1 extrahiert, wobei unter diesen Bedingungen nur der Ester in die organische Phase überging und der hydrolysierte Alkohol aufgrund des unterschiedlichen logP-Wertes (siehe Tabelle 1) in der wässrigen Phase verblieb.

Zur Isolierung des Esters **(-)-1** wurden die vereinigten organischen Phasen einmal mit 400 ml einer 1-molaren wässrigen Natriumcarbonatlösung gewaschen, über Natriumsulfat getrocknet. Nach destillativer Entfernung des Lösungsmittels wurden 30.4 g Rohprodukt (93 % der Theorie) bestehend aus (-)-(1 R, 3 S, 4 S)-Buttersäure- 3-dimethylaminomethyl-4-hydroxy-4-(3-methoxy-phenyl)-cyclohexyl ester **(-)-1** erhalten. Die Rohbase ([α]_{D}²²= -12.0 ° (c = 1.02, Methanol)) wurde in 300 ml eines Lösungsmittelgemisches bestehend aus Ethanol und 2-Butanon im Verhältnis 1 : 9 aufgenommen und mit 11.0 ml Trimethylchlorsilan und 1.57 ml Wasser versetzt. 3.6 g (10 % der Theorie) des Hydrochlorids mit einem ee-Wert von 4.8 % kristallisierten aus. Nach Abtrennung wurde die Mutterlauge eingeengt. Nach Freisetzen der Base mit Natriumcarbonat und Extraktion mit Essigsäureethylester, Trocknen über Natriumsulfat und destillativer Entfernung des Lösungsmittels konnten 23.9 g (73 % der Theorie) (-)-(1 R, 3 S, 4 S)-Buttersäure- 3-dimethylaminomethyl-4-hydroxy-4-(3-methoxy-phenyl)-cyclohexyl ester **(-)-1** mit einem ee-Wert von 100 % (bestimmt nach chiraler HPLC) erhalten werden. Hieraus konnte durch alkalische Esterhydrolyse mit Kaliumhydroxid in Ethanol (-)-(1 S, 2 S, 4 R)-2-Dimethylaminomethyl-1-(3-methoxy-phenyl)-cyclohexan-1,4-diol **(-)-2** in quantitativer Ausbeute erhalten werden (Organikumsvorschrift).

Zur Isolierung von (+)-(1 R, 2 R, 4 S)-2-Dimethylaminomethyl-1-(3-methoxy-phenyl)-cyclohexan-1,4-diol **(+)-2** wurde die wässrige Phase der Esterhydrolyse mit 2-molarer Salzsäure auf einen pH-Wert von 5.0 eingestellt. Die so eingestellte Lösung wurde bei einer Badtemperatur von 60 °C und einem Druck von 650 mbar bis 150 mbar vom Lösungsmittel befreit. Der Rückstand wurde dann mit 2-molarer wässriger Natriumcarbonatlösung auf einen pH-Wert von 10.0 eingestellt und dreimal mit jeweils 100 ml Essigsäureethylester extrahiert. Die vereinigten organischen Phasen wurden über Natriumsulfat getrocknet. Nach destillativer Entfernung des Lösungsmittels konnten 26.0 g (100 % der Theorie) Rohprodukt erhalten werden. Die Rohbase wurde in 270 ml eines Lösungsmittelgemisches bestehend aus Ethanol und 2-Butanon im Verhältnis 1 : 9 aufgenommen und mit 12.2 ml Trimethylchlorsilan und 1.73 ml Wasser versetzt, wobei das Hydrochlorid des (+)-(1 R, 2 R, 4 S)-2-Dimethylaminomethyl-1-(3-methoxy-phenyl)-cyclohexan-1,4-diols **(+)-2** in 78 %iger Ausbeute (23.1 g) mit einem ee-Wert von 96.3 % (nach chiraler HPLC) auskristallisierte ([α]_{D}²²= +36.5 ° (c = 1.06, Methanol)).

Die folgende Tabelle 1 zeigt die pka-Werte und logP-Werte der Verbindungen **1** und **2**.

**Tabelle 1:**

| pka-Werte und logP-Werte der Verbindungen **1** und **2.** | | |
|---|---|---|
| | Verbindung **1** (Ester) | Verbindung **2** (Alkohol) |
| pKa-Wert | 8.796 | 9.055 |
| logP-Wert: Wasser/Octanol | 2.898 | 1.101 |
| logP-Wert: Wasser/Cyclohexan | 2.360 | -0.632 |
| logD-Wert bei pH 7.4 Wasser/Octanol | 1.484 | -0.564 |
| logD-Wert bei pH 7.4 Wasser/Cyclohexan | 0.946 | -2.297 |
| □logP-Wert bei pH 7.4 | 0.538 | 1.733 |
| □logD-Wert bei pH 7.4 | 0.538 | 1.733 |

Die Abhängigkeit des ee-Wertes von Ester und Alkohol in Abhängigkeit von der Reaktionszeit zeigt exemplarisch folgende Tabelle 2.

**Tabelle 2:**

| Abhängigkeit des ee-Wertes von Ester und Alkohol in Abhängigkeit von der Reaktionszeit (Gehalt und ee-Wert der Verbindungen **1** und **2** wurden mittels chiraler HPLC bestimmt): | | | | |
|---|---|---|---|---|
| Zeit in Stunden | Gehalt Ester in %¹⁾ | Gehalt (+)-Ester²⁾ (in %) | Gehalt (-)-Ester²⁾ (in %) | %-ee-Wert des (-)-Esters³⁾ |
| 3 | 91.2 | 40.8 | 50.4 | 10.5 |
| 19 | 68.6 | 17.7 | 50.9 | 48.4 |
| 24 | 64.4 | 14.0 | 50.4 | 56.6 |
| 28 | 62.2 | 11.4 | 50,9 | 63.4 |

| | Gehalt Alkohol in %¹⁾ | Gehalt (-)-Alkohol²⁾ (in %) | Gehalt (+)-Alkohol²⁾ (in %) | %-ee-Wert des (+)-Alkohol²⁾ |
|---|---|---|---|---|
| 3 | 8.8 | 0.4 | 8.4 | 91.6 |
| 19 | 31.4 | 0.5 | 30.9 | 96.6 |
| 24 | 35.6 | 0.7 | 35.0 | 96.2 |
| 28 | 37.8 | 0.7 | 37.1 | 96.5 |

| | | | | |
|---|---|---|---|---|
| 1) prozentuale Gehalt Ester bzw. Alkohol bezieht sich auf den bestimmten Gesamtgehalt Ester und Alkohol im Reaktionsgemisch; | | | | |
| 2) der prozentuale Gehalt der enantiomeren Ester bzw. Alkohole bezieht sich auf den Anteil Ester bzw. Alkohol im Gesamtgemisch ((+)-Enantiomer (Ester) + (-)-Enantiomer (Ester) + (+)-Enantiomer (Alkohol) + (-)-Enantiomer (Alkohol) = 100 %; | | | | |
| 3) der prozentuale ee-Wert wurde nach folgender Gleichung bestimmt: %-Überschussenantiomer - %-Unterschussenantiomer / %-Überschussenantiomer + %-Unterschussenantiomer. | | | | |

Die Abhängigkeit des %-ee-Wertes des Alkohols **(+)-2** von der zugesetzten Acetonmenge wird in Tabelle 3 gezeigt:

**Tabelle 3:**

| Abhängigkeit des %-ee-Wertes des Alkohols **(+)-2** von der zugesetzten Acetonmenge (1.5 mmol Ester **rac-1** als Hydrochlorid wurden in 5 ml Phosphatpuffer pH 7.0 (Fa. Merck) gelöst und mit 1.2 ml einer 1-molaren wässrigen Natriumhydrogencarbonatlösung versetzt; die zugesetzte Enzymmenge betrug 5.0 mg Chirazyme E1 der Fa. Roche Diagnostics; es wurde jeweils 19 Stunden bei Raumtemperatur gerührt; die Aufarbeitunng erfolgte wie in Beispiel 1 beschrieben) | | | | |
|---|---|---|---|---|
| Zusatz von Aceton (ml, %) | GesamtgehaltE ster¹⁾ (in %) | %-ee-Wert des (-)-Esters²⁾ | Gesamrgehalt Alkohol¹⁾ (in %) | %-ee-Wert des (+)-Alkohol²⁾ |
| 0 ml, 0 % | 46.1 | 90.7 | 53.9 | 72.5 |
| 0.6 ml, 9 % | 50.8 | 97.6 | 49.2 | 89.8 |
| 1.0 ml, 13.5 % | 56.8 | 85.7 | 43.3 | 96.9 |
| 1.2 ml 16% | 55.6 | 94.2 | 44.4 | 95.5 |

| | | | | |
|---|---|---|---|---|
| 1) der prozentuale Gehalt der enantiomeren Ester bzw. Alkohole bezieht sich auf den Anteil Ester bzw. Alkohol im Gesamtgemisch ((+)-Enantiomer (Ester) + (-)-Enantiomer (Ester) zu (+)-Enantiomer (Alkohol) + (-)-Enantiomer (Alkohol); | | | | |
| 2) der prozentuale ee-Wert wurde nach folgender Gleichung bestimmt: %-Überschussenantiomer - %-Unterschussenantiomer / %-Überschussenantiomer + %-Unterschussenantiomer. | | | | |

### Beispiel 4:

### Enzymatische Esterhydrolyse

### Lipase-katalysierte Hydrolyse des (1 SR, 3 RS, 4 RS)-Buttersäure- 3-dimethylaminomethyl-4-hydroxy-4-(3-methoxy-phenyl)-cyclohexyl esters (rac-1)

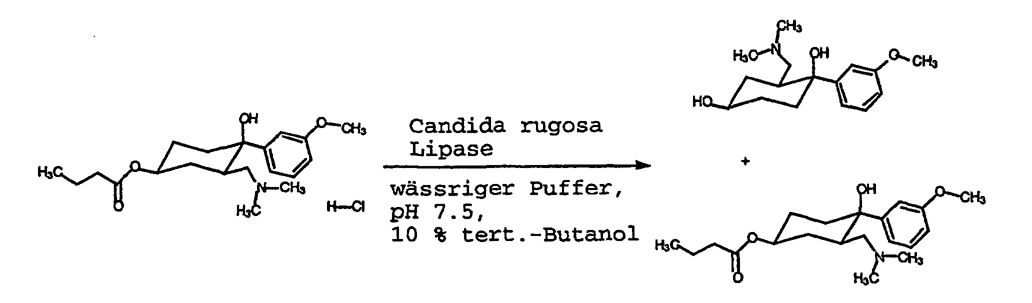

(+)-(1 S, 3 R, 4 R)-Buttersäure- 3-dimethylaminomethyl-4-hydroxy-4-(3-methoxyphenyl)-cyclohexyl ester (**(+)-1**)
und
(-)-(1 S, 2 S, 4 R)-2-Dimethylaminomethyl-1-(3-methoxy-phenyl)-cyclohexan-1,4-diol (**(-)-2**)

Analog wie in Beispiel 3 beschrieben führt die enzymatische Hydrolyse von **rac-1** unter Verwendung der Lipase *Candida rugosa* (Fa. Fluka) im wässrigen Puffersystem bei einem pH-Wert von 7.5 unter Verwendung von 10 % tert.-Butanol nach 24 Stunden Reaktionszeit bei Raumtemperatur zu einer entgegengesetzten asymmetrischen Induktion. Es konnten nach einem Umsatz von 28 % der Alkohol **(-)-2** mit einem ee-Wert von 89 % und der Ester **(+)-1** mit einem ee-Wert von 37 % isoliert werden (E=24).

### Beispiel 5:

### Enzymatische Esterhydrolyse

### Schweineleberesterase-katalysierte Hydrolyse des (1 SR, 3 RS, 4 RS, Buttersäure- 4-dimethylaminomethyl-3-hydroxy-3-(3-methoxy-phenyl)-cyclohexyl ester, Hydrochlorids (rac-3)

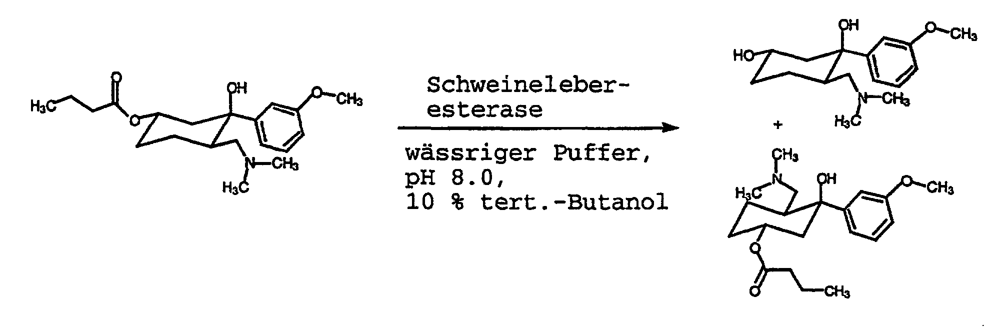

(+)-(1 R, 3 S, 6 R)-6-Dimethylaminomethyl-1-(3-methoxy-phenyl)-cyclohexan-1,3-diol (**(+)-4**)
und
(-)-(1 R, 3 S, 4 S)- Buttersäure- 4-dimethylaminomethyl-3-hydroxy-3-(3-methoxyphenyl)-cyclohexyl ester (**(-)-3)**

Analog wie in Beispiel 3 beschrieben führt die enzymatische Hydrolyse von rac-3 unter Verwendung der Schweineleberesterase im wässrigen Puffersystem bei einem pH-Wert von 8.0 unter Verwendung von 10 % tert.-Butanol nach 6 Stunden Reaktionszeit bei Raumtemperatur zu einem 40 %igem Umsatz. Auf diese Weise konnten in 79 % Ausbeute der Ester **(-)-3** mit einem ee-Wert von 86 % ([α]_{D}²²= -6.0 ° (c = 0.81, Methanol)) und der Alkohol (+)-4 in 77 % Ausbeute und einem ee-Wert von 94 % ([α]_{D}²²= +21.7° (c = 0.80, Methanol)) erhalten werden (E=46).

### Beispiel 6:

### Enzymatische Esterhydrolyse

### Lipase-katalysierte Hydrolyse des (1 SR, 3 RS, 4 RS, Buttersäure- 4-dimethylaminomethyl-3-hydroxy-3-(3-methoxy-phenyl)-cyclohexyl ester, Hydrochlorids (rac-3)

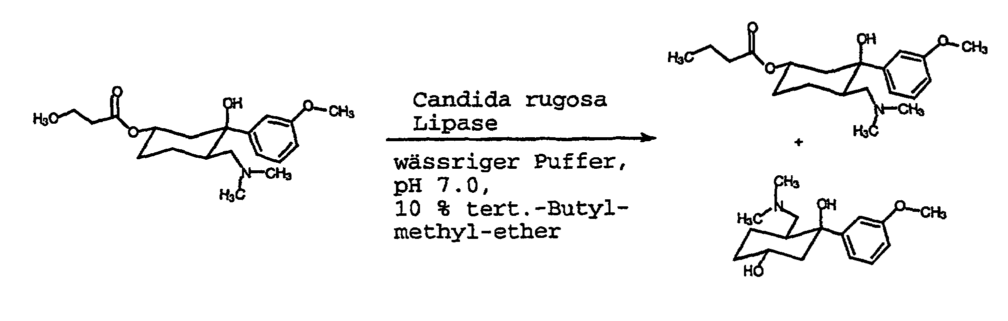

(-)-(1 S, 3 R, 6 S)-6-Dimethylaminomethyl-1-(3-methoxy-phenyl)-cyclohexan-1,3-diol (**(-)-4)**
und
(+)-(1 S, 3 R, 4 R)- Buttersäure- 4-dimethylaminomethyl-3-hydroxy-3-(3-methoxyphenyl)-cyclohexyl ester (**(+)-3**)

Analog wie in Beispiel 3 beschrieben führt die enzymatische Hydrolyse von **rac-3** unter Verwendung der Lipase *Candida rugosa* im wässrigen Puffersystem bei einem pH-Wert von 7.0 unter Verwendung von 10 % tert.-Butanylmethylether nach 6 Stunden Reaktionszeit bei Raumtemperatur zu einem 45 %igem Umsatz. Auf diese Weise konnten in 80 % Ausbeute der Ester **(+)-3** mit einem ee-Wert von >99 % ([α]_{D}²²= + 7.5 ° (c = 0.74, Methanol)) und der Alkohol **(-)-4** in 79 % Ausbeute und einem ee-Wert von >99 % ([α]_{D}²²= -29.5 ° (c = 1.01, Methanol)) erhalten werden (E > 200).

### Beispiel 7:

### Enzymatische Transacylierung in organischen Lösungsmitteln

### Lipase-katalysierte Transacylierung des (1 RS, 3 SR, 6 RS)-6-Dimethylaminomethyl-1-(3-methoxy-phenyl)-cyclohexan-1,3-diols rac-4 mit verschiedenen Acylierungsreagenzien zu den Estern 5 und 6

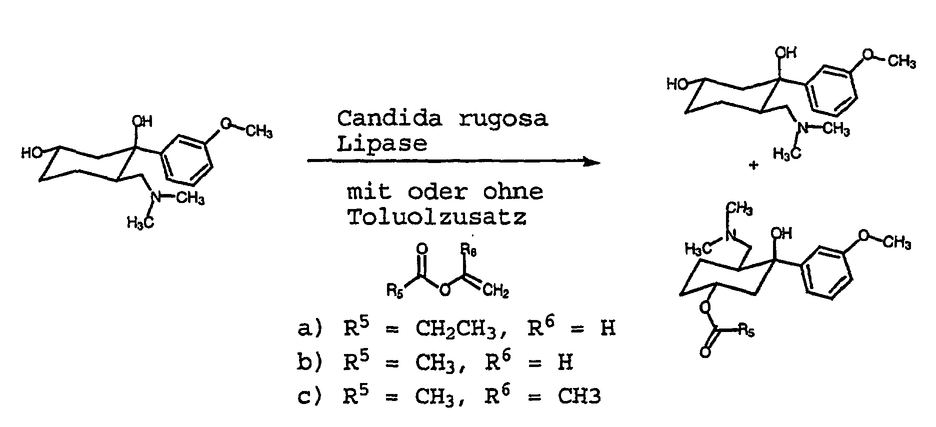

(1 R, 3 S, 6 R)-6-Dimethylaminomethyl-1-(3-methoxy-phenyl)-cyclohexan-1,3-diol (+)-4
und
R⁵ = CH₃: (-)-(1 R, 3 S, 4 S)-Essigsäure- 4-dimethylaminomethyl-3-hydroxy-3-(3-methoxy-phenyl)-cyclohexyl ester **(-)-5**
oder
R⁵ = CH₂CH₃: (-)-(1 R, 3 S, 4 S)-Propionsäure- 4-dimethylaminomethyl-3-hydroxy-3-(3-methoxy-phenyl)-cyclohexyl ester **(-)-6**

Zur Transacylierung wurden 70 mg (0.25 mmol) (1 RS, 3 SR, 6 RS)-6-Dimethylaminomethyl-1-(3-methoxy-phenyl)-cyclohexan-1,3-diol **rac-4** in einem Lösungsmittelgemisch bestehend aus Toluol und dem Transacylierungsreagenz oder unter Verwendung des Transacylierungmittels selbst als Lösungsmittel aufgenommen und zumächst zwei Stunden bei Raumtemperatur gerührt. Nach Zugabe der Lipase *Candida rugosa* (5 mg, 185 Units) wurde 5 bis 9 Tage bei Raumtemperatur gerührt. Zur Abtrennung des Enzyms wurde über Kieselgel filtriert. Alkohol und Ester wurden wie in Beispiel 1 beschrieben voneinander getrennt und isoliert. Die Ergebnisse sind in Tabelle 3 zusammengefasst.

Anstelle der Lipase *Candida rugosa* wurden in analoger Weise auch die Lipasen *Candida cylindracea* oder *Pseudomonas cepacia* eingesetzt.

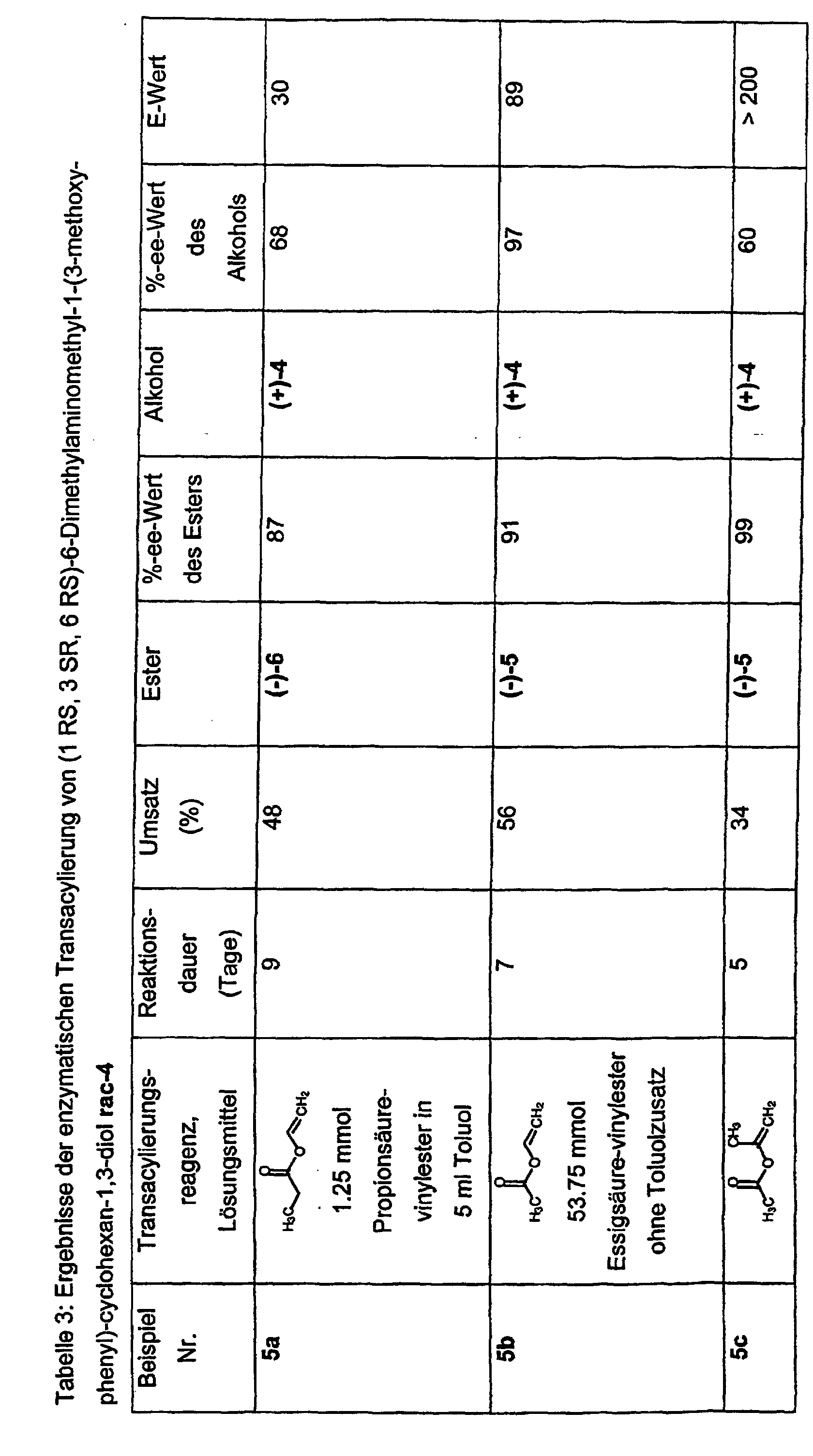

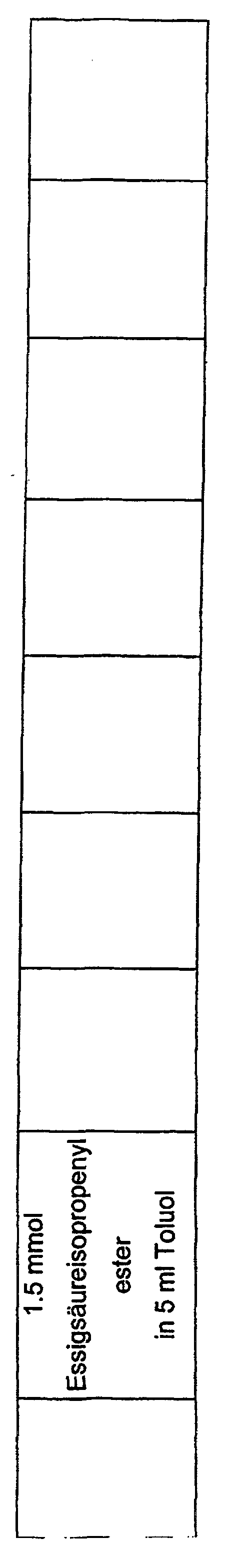

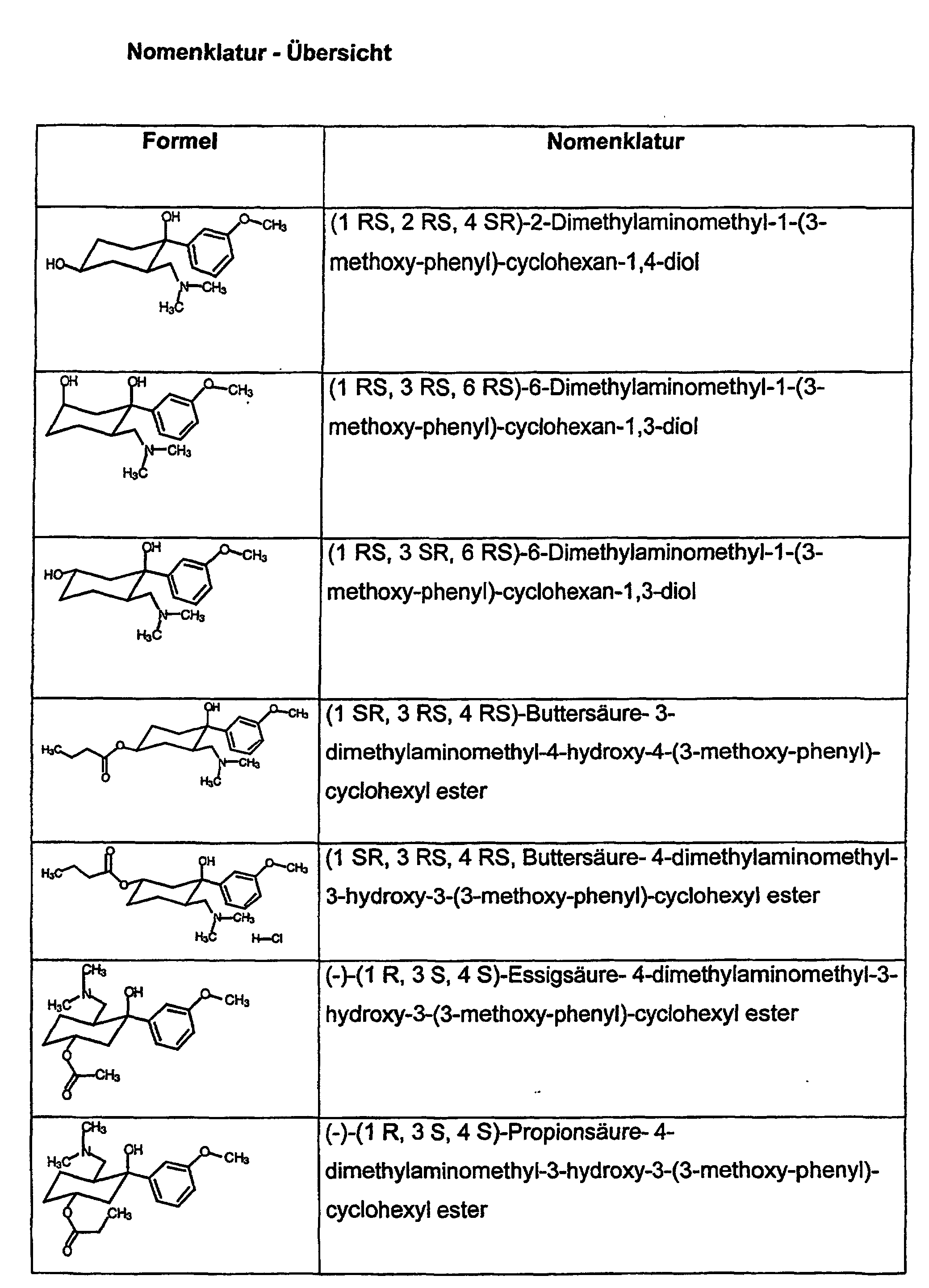

## Patentansprüche

1. Verfahren zur enzymatischen Racematspaltung von Aminomethyl-Aryl-Cyclohexanol-Derivaten der allgemeinen Formel I 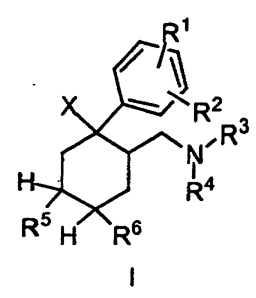 , worin X ausgewählt ist aus
H, F, Cl, Br, I, CF₃, O-S(O₂)-C₆H₄-pCH₃, OR¹⁴ oder OC(O)R¹⁴, wobei R¹⁴ ausgewählt ist aus
H; C₁-C₁₀-Alkyl, C₂-C₁₀-Alkenyl oder C₂-C₁₀-Alkinyl, jeweils verzweigt oder unverzweigt, einfach oder mehrfach substituiert oder unsubstituiert; C₃-C₇-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl oder Alkylheteroaryl, gesättigt oder ungesättigt, einfach oder mehrfach substituiert oder unsubstituiert; Aryl oder Heteroaryl, jeweils einfach oder mehrfach substituiert oder unsubstituiert;
R³, R⁴ unabhängig voneinander ausgewählt sind aus
H, C₁-C₁₀-Alkyl, C₂-C₁₀-Alkenyl oder C₂-C₁₀-Alkinyl, jeweils verzweigt oder unverzweigt, einfach oder mehrfach substituiert oder unsubstituiert; C₃-C₇-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl oder Alkylhereroaryl, gesättigt oder ungesättigt, einfach oder mehrfach substituiert oder unsubstituiert; Aryl oder Heteroaryl, jeweils einfach oder mehrfach substituiert oder unsubstituiert;
oder
R³ und R⁴ zusammen ein C₃-C₇-Cycloalkyl, gesättigt oder ungesättigt, einfach oder mehrfach substituiert oder unsubstituiert,bilden, bzw. einen entsprechenden Heterocyclus, bei dem ein C-Atom im Ring durch S, O oder NR¹⁵ ersetzt ist, mit R¹⁵ ausgewählt aus
H, C₁-C₁₀-Alkyl, C₂-C₁₀-Alkenyl oder C₂-C₁₀-Alkinyl, jeweils verzweigt oder unverzweigt, einfach oder mehrfach substituiert oder unsubstituiert;
R¹ und R² unabhängig voneinander ausgewählt sind aus R¹⁰ oder YR¹⁰ mit Y = C₁-C₁₀-Alkyl, C₂-C₁₀-Alkenyl oder C₂-C₁₀-Alkinyl, verzweigt oder unverzweigt, einfach oder mehrfach substituiert oder unsubstituiert, wobei R¹⁰ ausgewählt ist aus
H, F, Cl, Br, I, CN, NO₂, C₁-C₈-Alkyl, C₂-C₈-Alkenyl oder C₂-C₈-Alkinyl, jeweils verzweigt oder unverzweigt, einfach oder mehrfach substituiert oder unsubstituiert; C₃-C₇-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder N ersetzt ist; Aryl oder Heteroaryl, jeweils einfach oder mehrfach substituiert oder unsubstituiert;
OR¹¹, OC(O)R¹¹, OC(O)OR¹¹, OC(S)R¹¹, C(O)R¹¹, C(O)OR¹¹, C(S)R¹¹, C(S)OR¹¹, SR¹¹, S(O)R¹¹ bzw. S(O₂)R¹¹, wobei R¹¹ ausgewählt ist aus
H, C₁-C₁₈-Alkyl, C₂-C₁₈-Alkenyl oder C₂-C₁₈-Alkinyl, jeweils verzweigt oder unverzweigt, einfach oder mehrfach substituiert oder unsubstituiert; C₃-C₇-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder N ersetzt ist; Alkylaryl oder Alkylheteroaryl, gesättigt oder ungesättigt, einfach oder mehrfach substituiert oder unsubstituiert; Aryl oder Heteroaryl, jeweils einfach oder mehrfach substituiert oder unsubstituiert; oder
NR¹²R¹³, C(O)NR¹²R¹³ oder S(O₂)NR¹²R¹³, wobei R¹² und R¹³ unabhängig voneinander ausgewählt sind aus
H, C₁-C₁₈-Alkyl, C₂-C₁₈-Alkenyl oder C₂-C₁₈-Alkinyl, jeweils verzweigt oder unverzweigt, einfach oder mehrfach substituiert oder unsubstituiert; C₃-C₇-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder N ersetzt ist; Alkylaryl oder Alkylheteroaryl, gesättigt oder ungesättigt, einfach oder mehrfach substituiert oder unsubstituiert; Aryl oder Heteroaryl, jeweils einfach oder mehrfach substituiert oder unsubstituiert;
oder
R¹² und R¹³ zusammen ein C₃-C₇-Cycloalkyl, gesättigt oder ungesättigt, einfach oder mehrfach substituiert oder unsubstituiert, bilden, bzw. einen entsprechenden Heterocyclus, bei dem ein C-Atom im Ring durch S, O oder N ersetzt ist;
oder
R¹ und R² zusammen -CH=CH-CH=CH- bilden, wobei das entstehende Naphthylsystem ein- oder mehrfach substituiert sein kann.
und
jeweils einer von den Substituenten R⁵ und R⁶ H und der andere OH entspricht, **dadurch gekennzeichnet, daß** je nach gewünschtem Enantiomer der Aminomethyl-Aryl-Cyclohexanol-Derivate der allgemeinen Formel I
**entweder** in der Reaktionsaltemative I
das Racemat von Verbindungen gemäß Formel I zunächst verestert und anschließend enzymatisch transformiert wird und die entstehenden enantiomerenreinen Verbindungen getrennt werden
**oder** in der Reaktionsaltemative II
das Racemat von Verbindungen gemäß Formel I in Gegenwart eines Esters enzymatisch transformiert wird und die entstehenden enantiomerenreinen Verbindungen getrennt werden.

2. Verfahren gemäß Anspruch 1, **dadurch gekennzeichnet, daß** man in der Reaktionsalternative I eine racemische Verbindung gemäß Formel II, 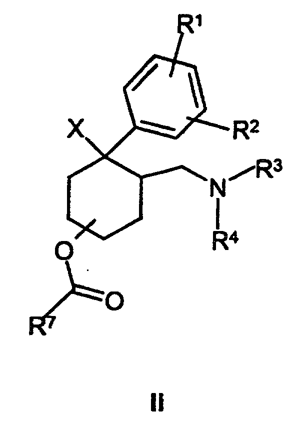 in der der Substituent OC(O)R⁷ der Position von R⁵ oder R⁶ in Formel I entspricht und R⁷ ausgewählt ist aus C₁-C₆-Alkyl, unsubstituiert oder einfach oder mehrfach substituiert; als freie Base oder in Form ihres Salzes in einem Lösungsmittel mit einer Lipase oder Esterase enzymatisch transformiert und die entstehende enantiomerenreinen Verbindungen gemäß Formeln III und Ia, 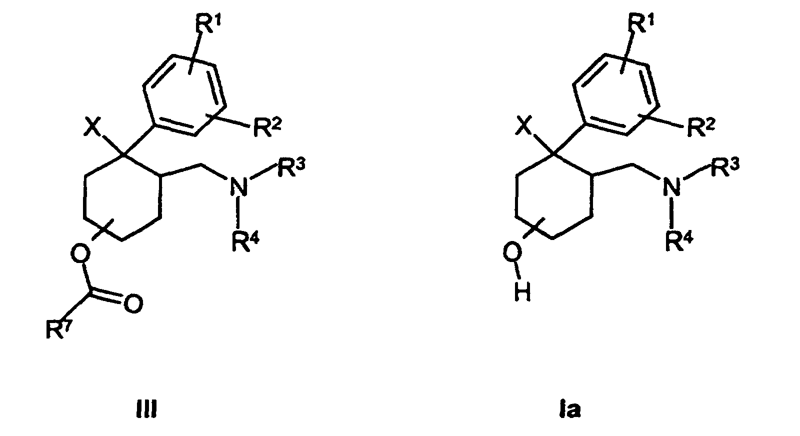 wobei Verbindungen nach Formel la Verbindungen nach Formel I entsprechen und der Substituent OH der Position von R⁵ oder R⁶ in Formel I entspricht,trennt.

3. Verfahren nach Anspruch 2, **dadurch gekennzeichnet, daß** R⁷ Chloracetyl-, Butyl- oder Pentyl- ist.

4. Verfahren nach einem der Ansprüche 2 oder 3, **dadurch gekennzeichnet, daß** das verwendete Enzym eine Esterase, vorzugsweise eine Schweineleberesterase ist.

5. Verfahren nach einem der Ansprüche 2 bis 4, **dadurch gekennzeichnet, daß** als Lösungsmittel ein wässriges Puffersystem, vorzugsweise mit einem pH zwischen 6,0 und 8,0 - vorzugsweise einem pH zwischen 7,0 und 7,5 - verwendet wird.

6. Verfahren nach einem der Ansprüche 2 bis 5, **dadurch gekennzeichnet, daß** als Lösungsmittel ein wässriges Puffersystem, vorzugsweise mit einem für das verwendete Enzym physiologischen pH verwendet wird.

7. Verfahren nach einem der Ansprüche 5 oder 6, **dadurch gekennzeichnet, daß** dem wäßrigen Puffersystem ein oder mehrere organische/s Lösungsmittel, vorzugsweise Aceton oder Butanol, bis zu einem Volumenprozentanteil zwischen 1 und 50%, vorzugsweise 5 und 20 %, zugefügt ist/sind.

8. Verfahren nach einem der Ansprüch 2 bis 7, **dadurch gekennzeichnet, daß** die Verbindung gemäß Formel II als Hydrochloridsalz eingesetzt wird.

9. Verfahren nach einem der Ansprüche 2 bis 8, **dadurch gekennzeichnet, daß** die eingesetzten Verbindungen gemäß Formel II dadurch hergestellt werden, daß racemische Verbindungen gemäß Formel I 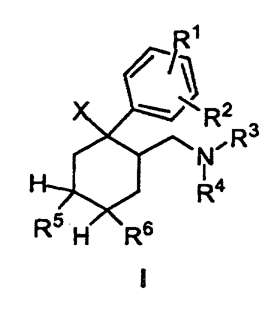 mit Basen, vorzugsweise Kalium-tert-butylat oder Natriumhydrid, in einem Lösungsmittel, vorzugsweise Tetrahydrofuran oder Dimethylformamid, in die Alkoholate überführt werden und anschließend unter Zugabe der entsprechenden Säurehalogenide in die racemischen Ester gemäß Formel II, 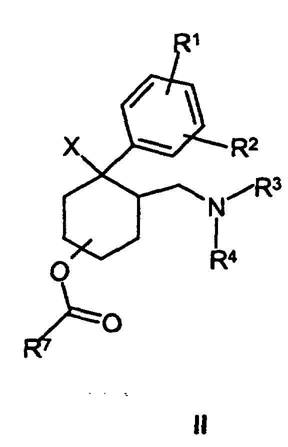 in denen der Substituent OC(O)R⁷ der Position von R⁵ oder R⁶ in Formel I entspricht, umgesetzt werden.

10. Verfahren gemäß Anspruch 1, **dadurch gekennzeichnet, daß** man in der Reaktionsalternative II eine racemische Verbindung gemäß Formel I 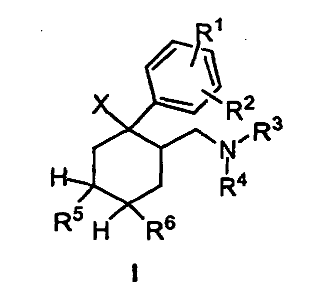 als freie Base oder in Form ihres Salzes in einem Lösungsmittel mit einem Ester gemäß Formel IV, 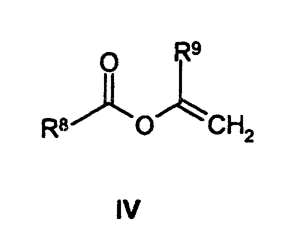 worin unabhängig voneinander R⁸ C₁-C₆-Alkyl, substituiert oder unsubstituiert; und R⁹ H oder C₁-C₆-Alkyl, substituiert oder unsubstituiert bedeuten, einsetzt, mit einer Lipase oder Esterase enzymatisch transformiert und die entstehenden enantiomerenreinen Verbindungen gemäß Formeln V und Ib, 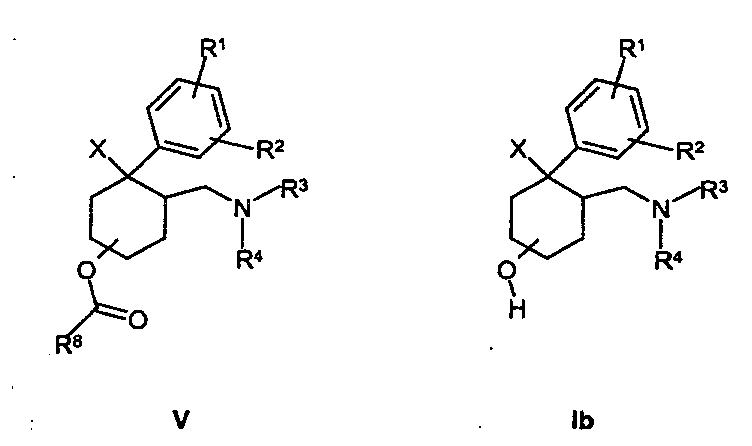 wobei Verbindungen nach Formel Ib Verbindungen nach Formel I entsprechen und der Substituent OH der Position von R⁵ oder R⁶ in Formel I entspricht, trennt.

11. Verfahren nach Anspruch 10, **dadurch gekennzeichnet, daß** in den Estern gemäß Formeln IV und V R⁸ Methyl oder Ethyl und/oder R⁹ gemäß Formel IV H oder Methyl bedeuten.

12. Verfahren nach einem der Ansprüche 10 oder 11, **dadurch gekennzeichnet, daß** der Ester gemäß Formel IV Vinylpropionat, Vinylacetat oder Isopropenylacetat ist.

13. Verfahren nach einem der Ansprüche 10 bis 12, **dadurch gekennzeichnet, daß** das verwendete Enzym eine Lipase, vorzugsweise eine Lipase aus *candida rugosa, Candida cylindracea* oder *Pseudomonas cepacia* ist.

14. Verfahren nach einem der Ansprüche 10 bis 13, **dadurch gekennzeichnet, daß** als Lösungsmittel ein organisches Lösungsmittel, vorzugsweise Toluol, verwendet wird.

15. Verfahren nach einem der Ansprüche 1 bis 14, **dadurch gekennzeichnet, daß** die Ester/Alkoholgemische nach Abschluß der enzymatischen Transformation durch pH-selektive Extraktion getrennt werden.

16. Verfahren nach einem der Ansprüche 1 bis 15, **dadurch gekennzeichnet, daß** R¹ = R¹⁰, wobei R¹⁰ ausgewählt ist aus
H, F, Cl, Br, I, CF₃, NO₂, NH₂; C₁-C₄-Alkyl oder C₂-C₄-Alkenyl, verzweigt oder unverzweigt, einfach oder mehrfach substituiert oder unsubstituiert; OR¹¹, C(O)OR¹¹ bzw. SR¹¹ wobei R¹¹ ausgewählt ist aus
H; C₁-C₄-Alkyl, verzweigt oder unverzweigt, einfach oder mehrfach substituiert oder unsubstituiert; vorzugsweise H, CF₃ oder CH₃,
oder S(O₂)NR¹²R¹³, wobei R¹² und R¹³ unabhängig voneinander ausgewählt sind aus
H; C₁-C₄-Alkyl, verzweigt oder unverzweigt, einfach oder mehrfach substituiert oder unsubstituiert;
wobei insbesondere bevorzugt ist, daß R¹ ausgewählt ist aus
H, F, Cl, OH, CH₃, C₂H₅, C₂H₃, CF₃, SCH₃, OCF₃, OCH₃, OC₂H₅, C(O)OCH₃, C(O)OC₂H₅, vorzugsweise m-OCH₃.

17. Verfahren nach einem der Ansprüche 1 bis 16, **dadurch gekennzeichnet, daß** R² = R¹⁰, wobei R¹⁰ ausgewählt ist aus
H, F, Cl, Br, I, SCH₃; C₁-C₄-Alkyl, C₂-C₄-Alkenyl verzweigt oder unverzweigt, einfach oder mehrfach substituiert oder unsubstituiert, vorzugsweise CF₃; OR¹¹, mit R¹¹ ausgewählt aus C₁-C₄-Alkyl, verzweigt oder unverzweigt, einfach oder mehrfach substituiert oder unsubstituiert, vorzugsweise CH₃;
wobei insbesondere bevorzugt ist, daß R² = H.

18. Verfahren nach einem der Ansprüche 1 bis 17, **dadurch gekennzeichnet, daß** X ausgewählt ist aus
H, F, Cl, OH, CF₃, O-S(O₂)-C₆H₄-pCH₃ bzw. OC(O)R¹² mit R¹² = H; C₁-C₄-Alkyl oder C₂-C₄-Alkenyl, verzweigt oder unverzweigt, ein- oder mehrfach substituiert oder unsubstituiert,
vorzugsweise H, F, Cl, OH, O-S(O₂)-C₆H₄-pCH₃, OC(O)R¹² mit R¹² = C₁-C₄-Alkyl, vorzugsweise CH₃;
wobei insbesondere bevorzugt ist, daß X = OH, F oder Cl, vorzugsweise OH.

19. Verfahren nach einem der Ansprüche 1 bis 18, **dadurch gekennzeichnet, daß** R³ und R⁴ unabhängig voneinander ausgewählt sind aus
C₁-C₄-Alkyl, verzweigt oder unverzweigt, einfach oder mehrfach substituiert oder unsubstituiert, vorzugsweise CH₃,
oder
R³ und R⁴ zusammen ein C₃-C₇-Cycloalkyl, gesättigt oder ungesättigt, einfach oder mehrfach substituiert oder unsubstituiert, bilden,
wobei insbesondere bevorzugt ist, daß R³ und R⁴ jeweils CH₃ bedeuten.

20. Verbindung gemäß Formel II, 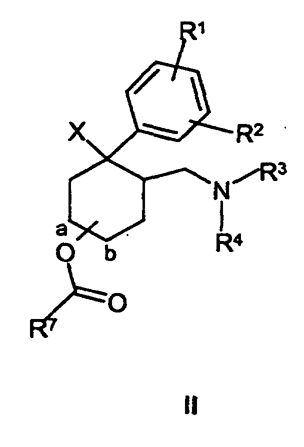 worin
X ausgewählt ist aus
H, F, Cl, Br, I, CF₃, O-S(O₂)-C₆H₄-pCH₃, OR¹⁴ oder OC(O)R¹⁴, wobei R¹⁴ ausgewählt ist aus
H; C₁-C₁₀-Alkyl, C₂-C₁₀-Alkenyl oder C₂-C₁₀-Alkinyl, jeweils verzweigt oder unverzweigt, einfach oder mehrfach substituiert oder unsubstituiert; C₃-C₇-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl oder Alkylheteroaryl, gesättigt oder ungesättigt, einfach oder mehrfach substituiert oder unsubstituiert; Aryl oder Heteroaryl, jeweils einfach oder mehrfach substituiert oder unsubstituiert;
R³, R⁴ unabhängig voneinander ausgewählt sind aus
H, C₁-C₁₀-Alkyl, C₂-C₁₀-Alkenyl oder C₂-C₁₀-Alkinyl, jeweils verzweigt oder unverzweigt, einfach oder mehrfach substituiert oder unsubstituiert; C₃-C₇-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl oder Alkylhereroaryl, gesättigt oder ungesättigt, einfach oder mehrfach substituiert oder unsubstituiert; Aryl oder Heteroaryl, jeweils einfach oder mehrfach substituiert oder unsubstituiert;
oder
R³ und R⁴ zusammen ein C₃-C₇-Cycloalkyl, gesättigt oder ungesättigt, einfach oder mehrfach substituiert oder unsubstituiert, bilden, bzw. einen entsprechenden Heterocyclus, bei dem ein C-Atom im Ring durch S, O oder NR¹⁵ ersetzt ist, mit R¹⁵ ausgewählt aus
H, C₁-C₁₀-Alkyl, C₂-C₁₀-Alkenyl oder C₂-C₁₀-Alkinyl, jeweils verzweigt oder unverzweigt, einfach oder mehrfach substituiert oder unsubstituiert;
R¹ und R² unabhängig voneinander ausgewählt sind aus R¹⁰ oder YR¹⁰ mit Y = C₁-C₁₀-Alkyl, C₂-C₁₀-Alkenyl oder C₂-C₁₀-Alkinyl, verzweigt oder unverzweigt, einfach oder mehrfach substituiert oder unsubstituiert, wobei R¹⁰ ausgewählt ist aus
H, F, Cl, Br, I, CN, NO₂, C₁-C₈-Alkyl, C₂-C₈-Alkenyl oder C₂-C₈-Alkinyl, jeweils verzweigt oder unverzweigt, einfach oder mehrfach substituiert oder unsubstituiert; C₃-C₇-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder N ersetzt ist; Aryl oder Heteroaryl, jeweils einfach oder mehrfach substituiert oder unsubstituiert;
OR¹¹, OC(O)R¹¹, OC(O)OR¹¹, OC(S)R¹¹, C(O)R¹¹, C(O)OR¹¹, C(S)R¹¹, C(S)OR¹¹, SR¹¹, S(O)R¹¹ bzw. S(O₂)R¹¹, wobei R¹¹ ausgewählt ist aus
H, C₁-C₁₈-Alkyl, C₂-C₁₈-Alkenyl oder C₂-C₁₈-Alkinyl, jeweils verzweigt oder unverzweigt, einfach oder mehrfach substituiert oder unsubstituiert; C₃-C₇-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder N ersetzt ist; Alkylaryl oder Alkylheteroaryl, gesättigt oder ungesättigt, einfach oder mehrfach substituiert oder unsubstituiert; Aryl oder Heteroaryl, jeweils einfach oder mehrfach substituiert oder unsubstituiert; oder
NR¹²R¹³, C(O)NR¹²R¹³ oder S(O₂)NR¹²R¹³, wobei R¹² und R¹³ unabhängig voneinander ausgewählt sind aus
H, C₁-C₁₈-Alkyl, C₂-C₁₈-Alkenyl oder C₂-C₁₈-Alkinyl, jeweils verzweigt oder unverzweigt, einfach oder mehrfach substituiert oder unsubstituiert; C₃-C₇-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder N ersetzt ist; Alkylaryl oder Alkylheteroaryl, gesättigt oder ungesättigt, einfach oder mehrfach substituiert oder unsubstituiert; Aryl oder Heteroaryl, jeweils einfach oder mehrfach substituiert oder unsubstituiert;
oder
R¹² und R¹³ zusammen ein C₃-C₇-Cycloalkyl, gesättigt oder ungesättigt, einfach oder mehrfach substituiert oder unsubstituiert, bilden, bzw. einen entsprechenden Heterocyclus, bei dem ein C-Atom im Ring durch S, O oder N ersetzt ist;
oder
R¹ und R² zusammen -CH=CH-CH=CH- bilden, wobei das entstehende Naphthylsystem ein- oder mehrfach substituiert sein kann.
der Substituent OC(O)R⁷ an der Position a oder b mit dem Cyclohexanring gemäß Formel II verbunden ist
und
R⁷ ausgewählt ist aus C1-C6-Alkyl, unsubstituiert oder einfach oder mehrfach substituiert; als freie Base oder in Form ihres Salzes.

21. Verbindung nach Anspruch 20, **dadurch gekennzeichnet, daß** R¹ = R¹⁰, wobei R¹⁰ ausgewählt ist aus
H, F, Cl, Br, I, CF₃, NO₂, NH₂; C₁-C₄-Alkyl oder C₂-C₄-Alkenyl, verzweigt oder unverzweigt, einfach oder mehrfach substituiert oder unsubstituiert; OR¹¹, C(O)OR¹¹ bzw. SR¹¹ wobei R¹¹ ausgewählt ist aus
H; C₁-C₄-Alkyl, verzweigt oder unverzweigt, einfach oder mehrfach substituiert oder unsubstituiert; vorzugsweise H, CF₃ oder CH₃.
oder S(O₂)NR¹²R¹³, wobei R¹² und R¹³ unabhängig voneinander ausgewählt sind aus
H; C₁-C₄-Alkyl, verzweigt oder unverzweigt, einfach oder mehrfach substituiert oder unsubstituiert;
, wobei insbesondere bevorzugt ist, daß R¹ ausgewählt ist aus H, F, Cl, OH, CH₃, C₂H₅, C₂H₃, CF₃, SCH₃, OCF₃, OCH₃, OC₂H₅, C(O)OCH₃, C(O)OC₂H₅, vorzugsweise m-OCH₃.

22. Verbindung nach einem der Ansprüche 20 oder 21, **dadurch gekennzeichnet, daß** R² = R¹⁰, wobei R¹⁰ ausgewählt ist aus
H, F, Cl, Br, I, SCH₃; C₁-C₄-Alkyl, C₂-C₄-Alkenyl verzweigt oder unverzweigt, einfach oder mehrfach substituiert oder unsubstituiert, vorzugsweise CF₃; OR¹¹, mit R¹¹ ausgewählt aus C₁-C₄-Alkyl, verzweigt oder unverzweigt, einfach oder mehrfach substituiert oder unsubstituiert, vorzugsweise CH₃;
wobei insbesondere bevorzugt ist, daß R² = H.

23. Verbindung nach einem der Ansprüche 20 bis 22, **dadurch gekennzeichnet, daß** X ausgewählt ist aus
H, F, Cl, OH, CF₃, O-S(O₂)-C₆H₄-pCH₃ bzw. OC(O)R¹² mit R¹² = H; C₁-C₄-Alkyl oder C₂-C₄-Alkenyl, verzweigt oder unverzweigt, ein- oder mehrfach substituiert oder unsubstituiert,
vorzugsweise H, F, Cl, OH, O-S(O₂)-C₆H₄-pCH₃, OC(O)R¹² mit R¹² = C₁-C₄-Alkyl, vorzugsweise CH₃;
wobei insbesondere bevorzugt ist, daß X = OH, F oder Cl, vorzugsweise OH.

24. Verbindung nach einem der Ansprüche 20 bis 23, **dadurch gekennzeichnet, daß** R³ und R⁴ unabhängig voneinander ausgewählt sind aus
C₁-C₄-Alkyl, verzweigt oder unverzweigt, einfach oder mehrfach substituiert oder unsubstituiert, vorzugsweise CH₃,
oder
R³ und R⁴ zusammen ein C₃-C₇-Cycloalkyl, gesättigt oder ungesättigt, einfach oder mehrfach substituiert oder unsubstituiert, bilden,
wobei insbesondere bevorzugt ist, daß R³ und R⁴ jeweils CH₃ bedeuten.

25. Verbindung nach einem der Ansprüche 20 bis 24, **dadurch gekennzeichnet, daß** es sich um (1SR,3RS,4RS)-Buttersäure-3-dimethylaminomethyl-4-hydroxy-4-(3-methoxy-phenyl)-cyclohexylester, insbesondere das Hydrochlorid, handelt.

26. Verfahren zur Herstellung einer Verbindung gemäß einem der Ansprüche 20 bis 25, bei dem racemische Verbindungen gemäß Formel I 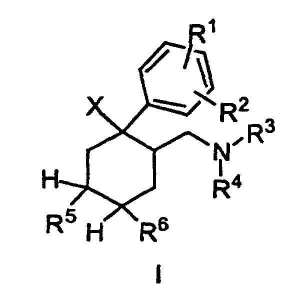 mit Basen, vorzugsweise Kalium-tert-butylat oder Natriumhydrid, in einem Lösungsmittel, vorzugsweise Tetrahydrofuran oder Dimethylformamid, in die Alkoholate überführt und anschließend unter Zugabe der entsprechenden Säurehalogenide in die racemischen Ester gemäß Formel II, 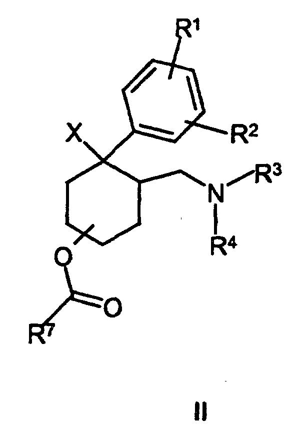 in denen der Substituent OC(O)R⁷ der Position von R⁵ oder R⁶ in Formel I entspricht, umgesetzt werden.

27. Arzneimittel enthaltend eine Verbindung nach einem der Ansprüche 20 bis 25 in Form ihrer Diastereomere oder Enantiomere sowie ihrer freien Base oder eines mit einer physiologisch verträglichen Säure gebildeten Salzes, insbesondere des Hydrochloridsalzes.

28. Verwendung einer Verbindung nach einem der Ansprüche 20 bis 25, in Form ihrer Diastereomere oder Enantiomere sowie ihrer freien Base oder eines mit einer physiologisch verträglichen Säure gebildeten Satzes, insbesondere des Hydrochloridsalzes, zur Herstellung eines Arzneimittels zur Behandlung von Schmerz, insbesondere Migräne, Akutschmerz sowie neuropathischem oder chronischem Schmerz, von inflammatorischen und allergischen Reaktionen, Depressionen, Drogen- und/oder Alkoholmißbrauch, Gastritis, cardiovaskulären Erkrankungen, Atemwegserkrankungen, Husten, seelischen Erkrankungen und/oder Epilepsie sowie insbesondere von Harninkontinenz, Juckreiz und/oder Diarrhoe.

## Claims

1. Process for the enzymatic cleavage of racemates of aminomethyl-aryl-cyclohexanol derivatives of the general formula I 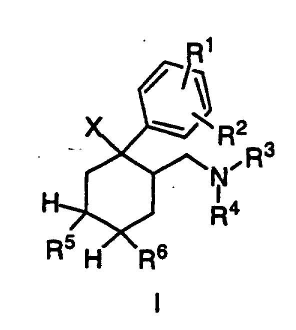 wherein X is chosen from
H, F, Cl, Br, I, CF₃, O-S(O₂)-C₆H₄-pCH₃, OR¹⁴ or OC(O)R¹⁴, wherein R¹⁴ is chosen from
H; C₁-C₁₀-alkyl, C₂-C₁₀-alkenyl or C₂-C₁₀-alkynyl, in each case branched or unbranched and mono- or polysubstituted or unsubstituted; C₃-C₇-cycloalkyl, saturated or unsaturated and mono- or polysubstituted or unsubstituted, or a corresponding heterocyclic radical in which a C atom in the ring is replaced by N, S or O; alkylaryl or alkylheteroaryl, saturated or unsaturated and mono- or polysubstituted or unsubstituted; aryl or heteroaryl, in each case mono- or polysubstituted or unsubstituted;
R³, R⁴ independently of one another are chosen from H, C₁-C₁₀-alkyl, C₂-C₁₀-alkenyl or C₂-C₁₀-alkynyl, in each case branched or unbranched and mono- or polysubstituted or unsubstituted; C₃-C₇ cycloalkyl, saturated or unsaturated and mono- or polysubstituted or unsubstituted, or a corresponding heterocyclic radical in which a C atom in the ring is replaced by N, S or O; alkylaryl or alkylhereroaryl [sic], saturated or unsaturated and mono- or polysubstituted or unsubstituted; aryl or heteroaryl, in each case monb- or polysubstituted or unsubstituted;
or
R³ and R⁴ together form a C₃-C₇-cycloalkyl, saturated or unsaturated and mono- or polysubstituted or unsubstituted, or a corresponding heterocyclic radical in which a C atom in the ring is replaced by S, O or NR¹⁵, where R¹⁵ is chosen from
H, C₁-C₁₀-alkyl, C₂-C₁₀-alkenyl or C₂-C₁₀-alkynyl, in each case branched or unbranched and mono- or polysubstituted or unsubstituted;
R¹ and R² independently of one another are chosen from R¹⁰ or YR¹⁰, where Y = C₁-C₁₀-alkyl, C₂-C₁₀-alkenyl or C₂-C₁₀-alkynyl, branched or unbranched and mono- or polysubstituted or unsubstituted, wherein R¹⁰ is chosen from
H, F, Cl, Br, I, CN, NO₂, C₁-C₈-alkyl, C₂-C₈-alkenyl or C₂-C₈-alkynyl, in each case branched or unbranched and mono- or polysubstituted or unsubstituted; C₃-C₇-cycloalkyl, saturated or unsaturated and mono- or polysubstituted or unsubstituted, or a corresponding heterocyclic radical in which a C atom in the ring is replaced by S, O or N; aryl or heteroaryl, in each case mono- or polysubstituted or unsubstituted;
OR¹¹, OC(O)R¹¹, OC(O)OR¹¹, OC(S)R¹¹, C(O)R¹¹, C(O)OR¹¹, C(S)R¹¹, C(S)OR¹¹, SR¹¹, S(O)R¹¹ or S(O₂)R¹¹, wherein R¹¹ is chosen from
H, C₁-C₁₈-alkyl, C₂-C₁₈-alkenyl or C₂-C₁₈-alkynyl, in each case branched or unbranched and mono- or polysubstituted or unsubstituted; C₃-C₇-cycloalkyl, saturated or unsaturated and mono- or polysubstituted or unsubstituted, or a corresponding heterocyclic radical in which a C atom in the ring is replaced by S, O or N; alkylaryl or alkylheteroaryl, saturated or unsaturated and mono- or polysubstituted or unsubstituted; aryl or heteroaryl, in each case mono- or polysubstituted or unsubstituted; or
NR¹²R¹³, C(O)NR¹²R¹³ or S(O₂)NR¹²R¹³, wherein R¹² and R¹³ independently of one another are chosen from
H, C₁-C₁₈-alkyl, C₂-C₁₈-alkenyl or C₂-C₁₈-alkynyl, in each case branched or unbranched and mono- or polysubstituted or unsubstituted; C₃-C₇-cycloalkyl, saturated or unsaturated and mono- or polysubstituted or unsubstituted, or a corresponding heterocyclic radical in which a C atom in the ring is replaced by S, O or N; alkylaryl or alkylheteroaryl, saturated or unsaturated and mono- or polysubstituted or unsubstituted; aryl or heteroaryl, in each case mono- or polysubstituted or unsubstituted;
or
R¹² and R¹³ together form a C₃-C₇-cycloalkyl, saturated or unsaturated and mono- or polysubstituted or unsubstituted, or a corresponding heterocyclic radical in which a C atom in the ring is replaced by S, O or N;
or
R¹ and R² together form -CH=CH-CH=CH-, wherein the naphthyl system formed can be mono- or polysubstituted.
and
in each case one of the substituents R⁵ and R⁶ corresponds to H and the other corresponds to OH, **characterized in that**, depending on the desired enantiomer of the aminomethyl-aryl-cyclohexanol derivatives of the general formula I
**either** in reaction alternative 1
the racemate of compounds according to formula I is first esterified and then transformed enzymatically and the enantiomerically pure compounds formed are separated
or in reaction alternative II
the racemate of compounds according to formula I is transformed enzymatically in the presence of an ester and the enantiomerically pure compounds formed are separated.

2. Process according to claim 1, **characterized in that** in reaction alternative 1 a racemic compound according to formula II 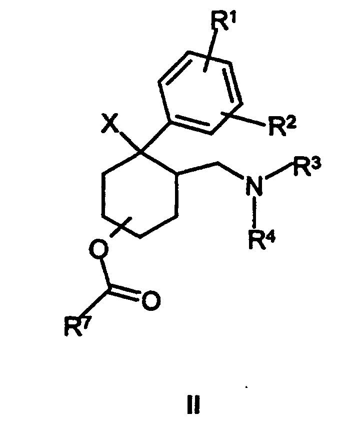 in which the substituent OC(O)R⁷ corresponds to the position of R⁵ or R⁶ in formula I and R⁷ is chosen from C₁-C₆-alkyl, unsubstituted or mono- or polysubstituted; as the free base or in the form of its salt, is transformed enzymatically in a solvent with a lipase or esterase and the enantiomerically pure compounds formed, according to formulae III and Ia 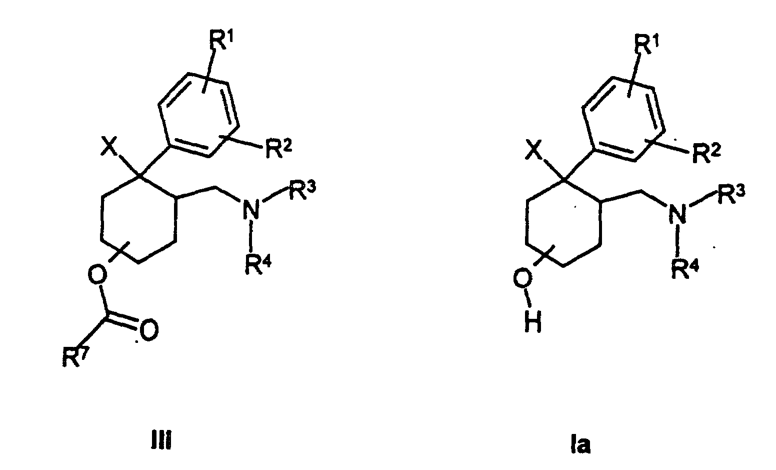 where compounds according to formula Ia correspond to compounds according to formula I and the substituent OR corresponds to the position of R⁵ or R⁶ in formula I, are separated.

3. Process according to claim 2, **characterized in that** R⁷ is chloroacetyl, butyl or pentyl.

4. Process according to one of claims 2 or 3, **characterized in that** the enzyme used is an esterase, preferably a pig liver esterase.

5. Process according to one of claims 2 to 4, **characterized in that** an aqueous buffer system, preferably with a pH of between 6.0 and 8.0 - preferably a pH of between 7.0 and 7.5 - is used as the solvent.

6. Process according to one of claims 2 to 5, **characterized in that** an aqueous buffer system, preferably with a physiological pH for the enzyme used, is used as the solvent.

7. Process according to one of claims 5 or 6, **characterized in that** one or more organic solvent(s), preferably acetone or butanol, is/are added to the aqueous buffer system up to a percentage content by volume of between 1 and 50%, preferably 5 and 20%.

8. Process according to one of claims 2 to 7,
**characterized in that** the compound according to formula II is employed as the hydrochloride salt.

9. Process according to one of claims 2 to 8,
**characterized in that** the compounds according to formula II employed are prepared by a process in which racemic compounds according to formula I 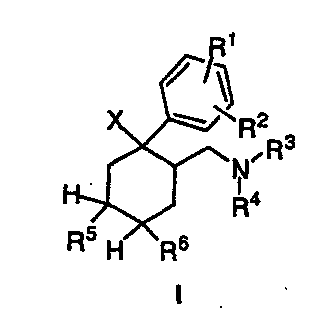 are converted with bases, preferably potassium tert-butylate or sodium hydride, in a solvent, preferably tetrahydrofuran or dimethylformamide, into the alcoholates and subsequently, with the addition of corresponding acid halides, into the racemic esters according to formula II 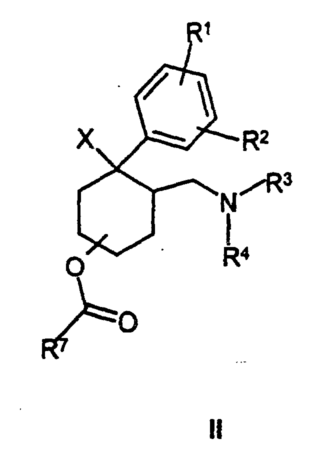 in which the substituent OH corresponds to the position of R⁵ or R⁶ in formula I.

10. Process according to claim 1, **characterized in that** in reaction alternative II a racemic compound according to formula I 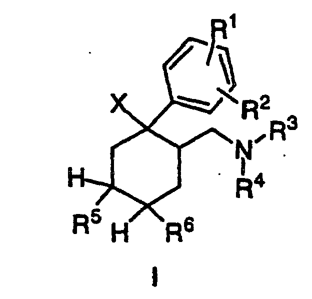 employed as the free base or in the form of its salt in a solvent with an ester according to formula IV 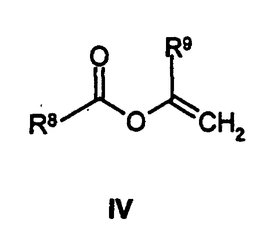 wherein, independently of one another, R⁸ denotes C₁-C₆-alkyl, substituted or unsubstituted; and R⁹ denotes H or C₁-C₆-alkyl, substituted or unsubstituted, is transformed enzymatically with a lipase or esterase and the enantiomerically pure compounds formed, according to formulae V and Ib 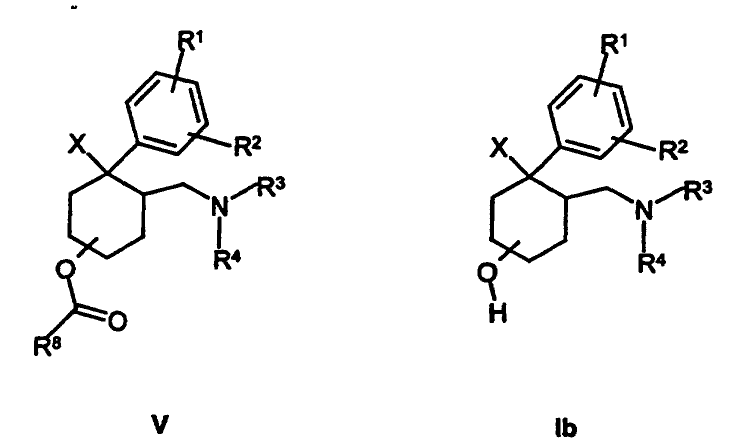 wherein compounds according to formula Ib correspond to compounds according to formula I and the substituent OH corresponds to the position of R⁵ or R⁶ in formula I, are separated.

11. Process according to claim 10, **characterized in that** in the esters according to formulae IV and V, R⁸ denotes methyl or ethyl and/or R⁹ according to formula IV denotes H or methyl.

12. Process according to one of claims 10 or 11, **characterized in that** the ester according to formula IV is vinyl propionate, vinyl acetate or isopropenyl acetate.

13. Process according to one of claims 10 to 12, **characterized in that** the enzyme used is a lipase, preferably a lipase from *candida* [sic] *rugosa, Candida cylindracea* or *Pseudomonas cepacia.*

14. Process according to one of claims 10 to 13, **characterized in that** an organic solvent, preferably toluene, is used as the solvent.

15. Process according to one of claims 1 to 14, **characterized in that** the ester/alcohol mixtures are separated by pH-selective extraction after conclusion of the enzymatic transformation.

16. Process according to one of claims 1 to 15, **characterized in that** R¹ = R¹⁰, wherein R¹⁰ is chosen from
H, F, Cl, Br, I, CF₃, NO₂, NH₂; C₁-C₄-alkyl or C₂-C₄-alkenyl, branched or unbranched and mono- or polysubstituted or unsubstituted; OR¹¹, C(O)OR¹¹ or SR¹¹, wherein R¹¹ is chosen from
H; C₁-C₄-alkyl, branched or unbranched and mono- or polysubstituted or unsubstituted; preferably H, CF₃ or CH₃,
or S(O₂)NR¹²R¹³, wherein R¹² and R¹³ independently of one another are chosen from
H; C₁-C₄-alkyl, branched or unbranched and mono- or polysubstituted or unsubstituted;
wherein R¹ is particularly preferably chosen from H, F, Cl, OH, CH₃, C₂H₅, C₂H₃, CF₃, SCH₃, OCF₃, OCH₃, OC₂H₅, C(O)OCH₃, C(O)OC₂H₅, preferably m-OCH₃.

17. Process according to one of claims 1 to 16, **characterized in that** R² = R¹⁰, wherein R¹⁰ is chosen from
H, F, Cl, Br, I, SCH₃; C₁-C₄-alkyl, C₂-C₄-alkenyl, branched or unbranched and mono- or polysubstituted or unsubstituted, preferably CF₃; OR¹¹, where R¹¹ is chosen from C₁-C₄-alkyl, branched or unbranched and mono- or polysubstituted or unsubstituted, preferably CH₃;
wherein R² particularly preferably = H.

18. Process according to one of claims 1 to 17,
haracterized in that X is chosen from
H, F, Cl, OH, CF₃, O-S(O₂)-C₆H₄-pCH₃ or OC(O)R¹² where R¹² = H; C₁-C₄-alkyl or C₂-C₄-alkenyl, branched or unbranched and mono- or polysubstituted or unsubstituted,
preferably H, F, Cl, OH, O-S(O₂)-C₆H₄-pCH₃, OC(O)R¹² where R¹² = C₁-C₄-alkyl, preferably CH₃;
wherein X particularly preferably = OH, F or Cl, preferably OH.

19. Process according to one of claims 1 to 18, **characterized in that** R³ and R⁴ independently of one another are chosen from
C₁-C₄-alkyl, branched or unbranched and mono- or polysubstituted or unsubstituted, preferably CH₃,
or
R³ and R⁴ together form a C₃-C₇-cycloalkyl, saturated or unsaturated and mono- or polysubstituted or unsubstituted,
wherein R³ and R⁴ particularly preferably in each case denote CH₃.

20. Compound according to formula II 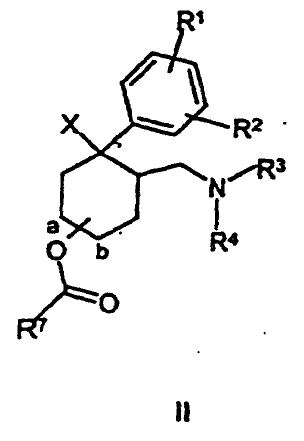 wherein
X is chosen from
H, F, Cl, Br, I, CF₃, O-S(O₂)-C₆H₄-pCH₃, OR¹⁴ or OC(O)R¹⁴, wherein R¹⁴ is chosen from
H; C₁-C₁₀-alkyl, C₂-C₁₀-alkenyl or C₂-C₁₀-alkynyl, in each case branched or unbranched and mono- or polysubstituted or unsubstituted; C₃-C₇-cycloalkyl, saturated or unsaturated and mono- or polysubstituted or unsubstituted, or a corresponding heterocyclic radical in which a C atom in the ring is replaced by N, S or O; alkylaryl or alkylheteroaryl, saturated or unsaturated and mono- or polysubstituted or unsubstituted; aryl or heteroaryl, in each case mono- or polysubstituted or unsubstituted;
R³, R⁴ independently of one another are chosen from
H, C₁-C₁₀-alkyl, C₂-C₁₀-alkenyl or C₂-C₁₀-alkynyl, in each case branched or unbranched and mono- or polysubstituted or unsubstituted; C₃-C₇cycloalkyl, saturated or unsaturated and mono- or polysubstituted or unsubstituted, or a corresponding heterocyclic radical in which a C atom in the ring is replaced by N, S or O; alkylaryl or alkylhereroaryl [sic], saturated or unsaturated and mono- or polysubstituted or unsubstituted; aryl or heteroaryl, in each case mono- or polysubstituted or unsubstituted;
or
R³ and R⁴ together form a C₃-C₇-cycloalkyl, saturated or unsaturated and mono- or polysubstituted or unsubstituted, or a corresponding heterocyclic radical in which a C atom in the ring is replaced by S, O or NR¹⁵, where R¹⁵ is chosen from
H, C₁-C₁₀-alkyl, C₂-C₁₀-alkenyl or C₂-C₁₀alkynyl, in each case branched or unbranched and mono- or polysubstituted or unsubstituted;
R¹ and R² independently of one another are chosen from R¹⁰ or YR¹⁰, where Y = C₁-C₁₀-alkyl, C₂-C₁₀-alkenyl or C₂-C₁₀-alkynyl, branched or unbranched and mono- or polysubstituted or unsubstituted, wherein R¹⁰ is chosen from
H, F, Cl, Br, I, CN, NO₂, C₁-C₈-alkyl, C₂-C₈alkenyl or C₂-C₈-alkynyl, in each case branched or unbranched and mono- or polysubstituted or unsubstituted; C₃-C₇cycloalkyl, saturated or unsaturated and mono- or polysubstituted or unsubstituted, or a corresponding heterocyclic radical in which a C atom in the ring is replaced by S, O or N; aryl or heteroaryl, in each case mono- or polysubstituted or unsubstituted;
OR¹¹, OC(O)R¹¹, OC(O)OR¹¹, OC(S)R¹¹, C(O)R¹¹, C(O)OR¹¹, C(S)R¹¹, C(S)OR¹¹, SR¹¹, S(O)R¹¹ or S(O₂)R¹¹, wherein R¹¹ is chosen from
H, C₁-C₁₈-alkyl, C₂-C₁₈-alkenyl or C₂-C₁₈alkynyl, in each case branched or unbranched and mono- or polysubstituted or unsubstituted; C₃-C₇-cycloalkyl, saturated or unsaturated and mono- or polysubstituted or unsubstituted, or a corresponding heterocyclic radical in which a C atom in the ring is replaced by S, O or N; alkylaryl or alkylheteroaryl, saturated or unsaturated and mono- or polysubstituted or unsubstituted; aryl or heteroaryl, in each case mono- or polysubstituted or unsubstituted; or
NR¹²R¹³, C(O)NR¹²R¹³ or S(O₂)NR¹²R¹³, wherein R¹² and R¹³ independently of one another are chosen from
H, C₁-C₁₈-alkyl, C₂-C₁₈-alkenyl or C₂-C₁₈-alkynyl, in each case branched or unbranched and mono- or polysubstituted or unsubstituted; C₃-C₇-cycloalkyl, saturated or unsaturated and mono- or polysubstituted or unsubstituted, or a corresponding heterocyclic radical in which a C atom in the ring is replaced by S, O or N; alkylaryl or alkylheteroaryl, saturated or unsaturated and mono- or polysubstituted or unsubstituted; aryl or heteroaryl, in each case mono- or polysubstituted or unsubstituted;
or
R¹² and R¹³ together form a C₃-C₇-cycloalkyl, saturated or unsaturated and mono- or polysubstituted or unsubstituted, or a corresponding heterocyclic radical in which a C atom in the ring is replaced by S, O or N;
or
R¹ and R² together form -CH=CH-CH=CH-, wherein the naphthyl system formed can be mono- or polysubstituted.
the substituent OC(O)R⁷ is bonded to the cyclohexane ring according to formula II at position a or b
and
R⁷ is chosen from C1-C6-alkyl, unsubstituted or mono- or polysubstituted; as the free base or in the form of its salt.

21. Compound according to claim 20, **characterized in that** R¹ = R¹⁰, wherein R¹⁰ is chosen from
H, F, Cl, Br, I, CF₃, NO₂, NH₂; C₁-C₄-alkyl or C₂-C₄-alkenyl, branched or unbranched and mono- or polysubstituted or unsubstituted; OR¹¹, C(O)OR¹¹ or SR¹¹, wherein R¹¹ is chosen from
H; C₁-C₄-alkyl, branched or unbranched and mono- or polysubstituted or unsubstituted; preferably H, CF₃ or CH₃,
or, S(O₂)NR¹²R¹³, wherein R¹² and R¹³ independently
of one another are chosen from
H; C₁-C₄-alkyl, branched or unbranched and mono- or polysubstituted or unsubstituted;
wherein R¹ is particularly preferably chosen from H, F, Cl, OH, CH₃, C₂H₅, C₂H₃, CF₃, SCH₃, OCF₃, OCH₃, OC₂H₅, C(O)OCH₃, C(O)OC₂H₅, preferably m-OCH₃.

22. Compound according to one of claims 20 or 21, **characterized in that** R² = R¹⁰, wherein R¹⁰ is chosen from
H, F, Cl, Br, I, SCH₃; C₁-C₄-alkyl, C₂-C₄-alkenyl, branched or unbranched and mono- or polysubstituted or unsubstituted, preferably CF₃; OR¹¹, where R¹¹ is chosen from C₁-C₄-alkyl, branched or unbranched and mono- or polysubstituted or unsubstituted, preferably CH₃;
wherein R² particularly preferably = H.

23. Compound according to one of claims 20 to 22,
**characterized in that** X is chosen from
H, F, Cl, OH, CF₃, O-S(O₂)-C₆H₄-pCH₃ or OC(O)R¹² where R¹² = H; C₁-C₄-alkyl or C₂-C₄-alkenyl, branched or unbranched and mono- or polysubstituted or unsubstituted,
preferably H, F, Cl, OH, O-S(O₂)-C₆H₄-pCH₃, OC(O)R¹² where R¹² = C₁-C₄-alkyl, preferably CH₃;
wherein X particularly preferably = OH, F or Cl, preferably OH.

24. Compound according to one of claims 20 to 23, **characterized in that** R³ and R⁴ independently of one another are chosen from
C₁-C₄-alkyl, branched or unbranched and mono- or polysubstituted or unsubstituted, preferably CH₃,
or
R³ and R⁴ together form a C₃-C₇-cycloalkyl, saturated or unsaturated and mono- or polysubstituted or unsubstituted,
wherein R³ and R⁴ particularly preferably in each case denote CH₃.

25. Compound according to one of claims 20 to 24, **characterized in that** it is (1SR,3RS,4RS)-butyric acid 3-dimethylaminomethyl-4-hydroxy-4-(3-methoxy-phenyl)-cyclohexyl ester, in particular the hydrochloride.

26. Process for the preparation of a compound according to one of claims 20 to 25, in which racemic compounds according to formula I 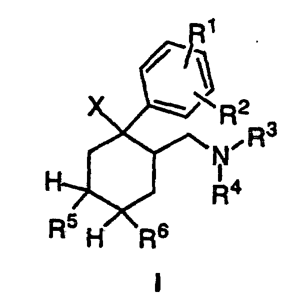 are converted with bases, preferably potassium tert-butylate or sodium hydride, in a solvent, preferably tetrahydrofuran or dimethylformamide, into the alcoholates and subsequently, with the addition of corresponding acid halides, into the racemic esters according to formula II 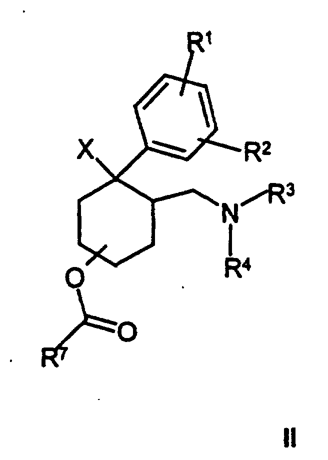 in which the substituent OC(O)R⁷ corresponds to the position of R⁵ or R⁶ in formula I.

27. Medicaments comprising a compound according to one of claims 20 to 25 in the form of its diastereomers or enantiomners and its free base or a salt formed with a physiologically acceptable acid, in particular the hydrochloride salt.

28. Use of a compound according to one of claims 20 to 25 in the form of its diastereomers or enantiomers and its free base or a salt formed with a physiologically tolerated acid, in particular the hydrochloride salt, for the preparation of a medicament for treatment of pain, in particular migraine, acute pain and neuropathic or chronic pain, of inflammatory and allergic reactions, depression, drug and/or alcohol abuse, gastritis, cardiovascular diseases, respiratory tract diseases, coughing, mental illnesses and/or epilepsy, and in particular of urinary incontinence, itching and/or diarrhoea.

## Revendications

1. Procédé de résolution enzymatique de racémates de dérivés d'aminométhyl-aryl-cyclohexanols de formule générale I 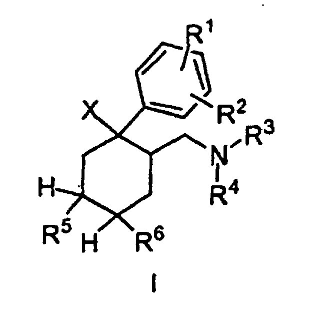 dans laquelle X est choisi entre
H, F, Cl, Br, I, CF₃, O-S(O₂)-C₆H₄-pCH₃, OR¹⁴ ou OC(O)R¹⁴, où R¹⁴ est choisi entre
H ; alkyle en C₁ à C₁₀, alcényle en C₂ à C₁₀ ou alcynyle en C₂ à C₁₀, chacun linéaire ou ramifié, substitué une ou plusieurs fois ou non substitué ; cycloalkyle en C₃ à C₇, saturé ou non saturé, substitué une ou plusieurs fois ou non substitué, ou un hétérocycle correspondant, dans lequel un atome de carbone du noyau est remplacé par N, S ou O ; alkylaryle ou alkylhétéroaryle, saturé ou non saturé, substitué une ou plusieurs fois ou non substitué ; aryle ou hétéroaryle, chacun substitué une ou plusieurs fois, ou non substitué ;
R³, R⁴ sont choisis indépendamment l'un de l'autre entre :
H, alkyle en C₁ à C₁₀, alcényle en C₂ à C₁₀ ou alcynyle en C₂ à C₁₀, chacun linéaire ou ramifié, substitué une ou plusieurs fois ou non substitué ; cycloalkyle en C₃ à C₇, saturé ou non saturé, substitué une ou plusieurs fois ou non substitué, ou un hétérocycle correspondant, dans lequel un atome de carbone du noyau est remplacé par N, S ou O ; alkylaryle ou alkylhétéroaryle, saturé ou non saturé, substitué une ou plusieurs fois ou non substitué ; aryle ou hétéroaryle, chacun substitué une ou plusieurs fois ou non substitué ;
ou bien
R³ et R⁴ forment ensemble un reste cycloalkyle en C₃ à C₇, saturé ou non saturé, substitué une ou plusieurs fois ou non substitué ; ou bien un hétérocycle correspondant, dans lequel un atome de carbone du noyau est remplacé par S, O ou NR¹⁵, R¹⁵ étant choisi entre
H, alkyle en C₁ à C₁₀, alcényle en C₂ à C₁₀ ou alcynyle en C₂ à C₁₀, chacun ramifié ou linéaire, substitué une ou plusieurs fois ou non substitué ;
R¹ et R² sont choisis indépendamment l'un de l'autre, entre R¹⁰ et YR¹⁰ avec Y = alkyle en C₁ à C₁₀, alcényle en C₂ à C₁₀ ou alcynyle en C₂ à C₁₀, ramifié ou linéaire, substitué une ou plusieurs fois ou non substitué, R¹⁰ étant choisi entre
H, F, Cl, Br, I, CN, NO₂, alkyle en C₁ à C₈, alcényle en C₂ à C₈ ou alcynyle en C₂ à C₈, chacun ramifié ou linéaire, substitué une ou plusieurs fois ou non substitué ; cycloalkyle en C₃ à C₇, saturé ou non saturé, substitué une ou plusieurs fois ou non substitué, ou un hétérocycle correspondant dans lequel un atome de carbone du noyau est remplacé par S, O ou N ; aryle ou hétéroaryle, chacun substitué une ou plusieurs fois ou non substitué ;
OR¹¹, OC(O)R¹¹, OC(O)OR¹¹, OC(S)R¹¹, C(O)R¹¹, C(O)OR¹¹, C(S)R¹¹, C(S)OR¹¹, SR¹¹, S(O)R¹¹ ou S(O₂)R¹¹, où R¹¹ est choisi entre
H, alkyle en C₁ à C₁₈, alcényle en C₂ à C₁₈ ou alcynyle en C₂ à C₁₈, chacun ramifié ou linéaire, substitué une ou plusieurs fois ou non substitué ; cycloalkyle en C₃ à C₇, saturé ou non saturé, substitué une ou plusieurs fois ou non substitué, ou un hétérocycle correspondant dans lequel un atome de carbone du noyau est remplacé par S, O ou N ; alkylaryle ou alkylhétéroaryle, saturé ou non saturé, substitué une ou plusieurs fois ou non substitué ; aryle ou hétéroaryle, chacun substitué une ou plusieurs fois ou non substitué ; ou bien
NR¹²R¹³, C(O)NR¹²R¹³ ou S(O₂)NR¹²R¹³, où R¹² et R¹³ sont choisis, indépendamment l'un de l'autre, entre
H ; alkyle en C₁ à C₁₈, alcényle en C₂ à C₁₈ ou alcynyle en C₂ à C₁₈, chacun linéaire ou ramifié, substitué une ou plusieurs fois ou non substitué ; cycloalkyle en C₃ à C₇, saturé ou non saturé, substitué une ou plusieurs fois ou non substitué, ou un hétérocycle correspondant, dans lequel un atome de carbone du noyau est remplacé par S, O ou N ; alkylaryle ou alkylhétéroaryle, saturé ou non saturé, substitué une ou plusieurs fois ou non substitué ; aryle ou hétéroaryle, chacun substitué une ou plusieurs fois ou non substitué ;
ou bien
R¹² et R¹³ forment ensemble un reste cycloalkyle en C₃ à C₇, saturé ou non saturé, substitué une ou plusieurs fois ou non substitué, ou un hétérocycle correspondant dans lequel un atome de carbone du noyau est remplacé par S, O ou N ;
ou bien
R¹ et R² forment ensemble un groupement -CH=CH-CH=CH-, le système naphtyle formé pouvant être substitué une ou plusieurs fois,
et
dans chaque cas l'un des substituants R⁵ et R⁶ correspond à H et l'autre correspond à OH, **caractérisé en ce que**, selon l'énantiomère souhaité des dérivés d'aminométhyl-arylcyclohexanols de formule générale I
dans la variante réactionnelle I
on estérifie tout d'abord le racémate de composés de formule I puis on transforme l'ester par voie enzymatique et on sépare les composés énantiomériquement purs formés
ou bien dans la variante réactionnelle II
on transforme par voie enzymatique le racémate de composés de formule I en présence d'un ester et on sépare les composés énantiomériquement purs formés.

2. Procédé suivant la revendication 1,
**caractérisé en ce que** dans la variante réactionnelle I, on transforme par voie enzymatique avec une lipase ou une estérase un composé racémique de formule II 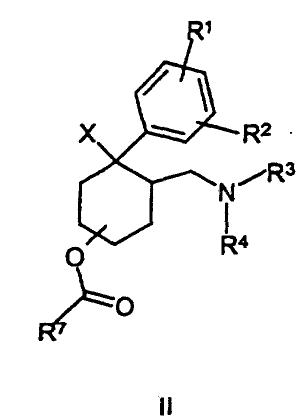 dans laquelle le substituant OC(O)R⁷ correspond à la position de R⁵ ou R⁶ dans la formule I et R⁷ est choisi entre un reste alkyle en C₁ à C₆ non substitué ou substitué une ou plusieurs fois ; à l'état de base libre ou sous forme de son sel dans un solvant et on sépare les composés énantiomériquement purs formés, répondant aux formules III et Ia 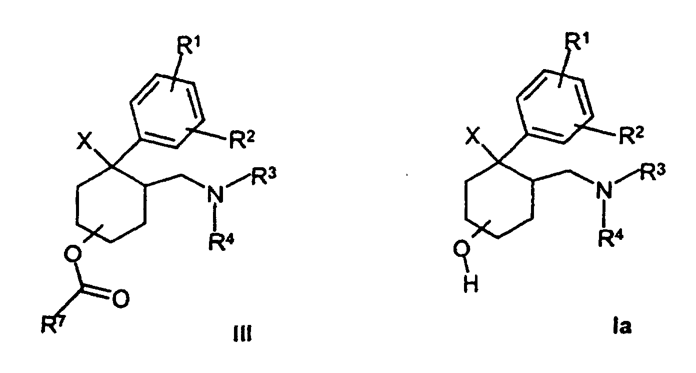 des composés de formule la correspondant à des composés de formule I et le substituant OH correspondant à la position de R⁵ ou de R⁶ dans la formule I.

3. Procédé suivant la revendication 2, **caractérisé en ce que** R⁷ est un reste chloracétyle, butyle ou pentyle.

4. Procédé suivant la revendication 2 ou 3, **caractérisé en ce que** l'enzyme utilisé est une estérase, avantageusement une estérase de foie de porc.

5. Procédé suivant l'une des revendications 2 à 4, **caractérisé en ce qu'**on utilise comme solvant un système tampon aqueux, ayant avantageusement un pH compris entre 6,0 et 8,0, notamment un pH compris entre 7,0 et 7,5.

6. Procédé suivant l'une des revendications 2 à 5, **caractérisé en ce qu'**on utilise comme solvant un système tampon aqueux, ayant avantageusement un pH physiologique pour l'enzyme utilisé.

7. Procédé suivant la revendication 5 ou 6, **caractérisé en ce qu'**on ajoute au système tampon aqueux un ou plusieurs solvants organiques, avantageusement de l'acétone ou du butanol, jusqu'à un pourcentage en volume compris entre 1 et 50 %, avantageusement entre 5 et 20 %.

8. Procédé suivant l'une des revendications 2 à 7, **caractérisé en ce qu'**on utilise le composé de formule II sous forme de son chlorhydrate.

9. Procédé suivant l'une des revendications 2 à 8, **caractérisé en ce que** les composés utilisés de formule II sont préparés par transformation des composés racémiques de formule I 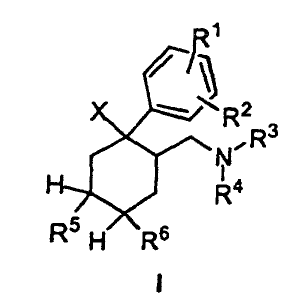 en alcoolates avec des bases, avantageusement du tertiobutylate de potassium ou de l'hydrure de sodium, dans un solvant, avantageusement le tétrahydrofuranne ou le diméthylformamide, puis transformation avec addition des halogénures d'acides correspondants en les esters racémiques de formule II 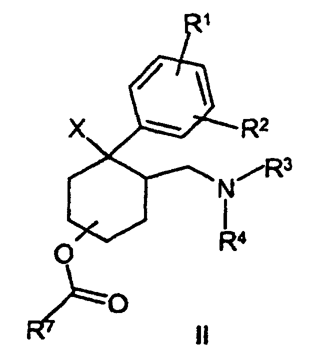 dans lesquels le substituant OC(O)R⁷ correspond à la position de R⁵ ou R⁶ dans la formule I.

10. Procédé suivant la revendication 1, **caractérisé en ce qu'**on utilise dans la variante réactionnelle II un composé racémique de formule I 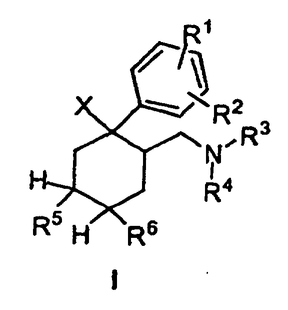 à l'état de base libre ou sous forme de son sel dans un solvant, avec un ester de formule IV, 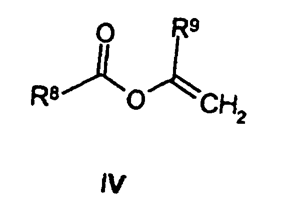 où indépendamment l'un de l'autre, R⁸ est un reste alkyle en C₁ à C₆ substitué ou non substitué et R⁹ représente H ou un reste alkyle en C₁ à C₆ substitué ou non substitué, on le transforme par voie enzymatique avec une lipase ou une estérase et on sépare les composés énantiomériquement purs obtenus de formules V et Ib, 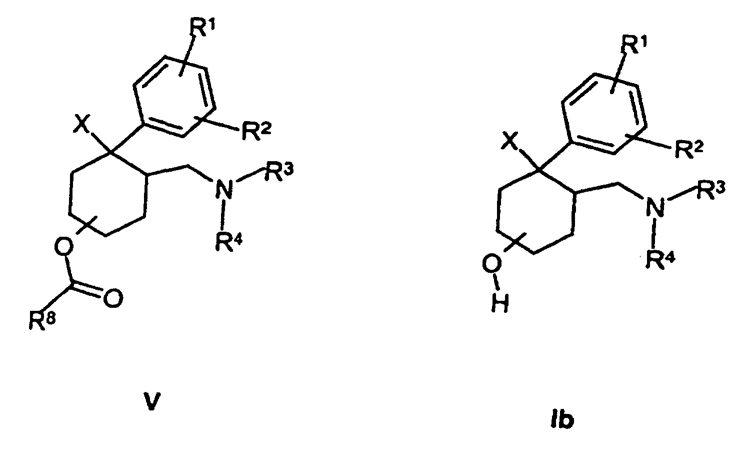 des composés de formule Ib correspondant à des composés de formule I et le substituant OH correspondant à la position de R⁵ ou R⁶ dans la formule I.

11. Procédé suivant la revendication 10, **caractérisé en ce que** dans les esters répondant aux formules IV et V, R⁸ désigne un reste méthyle ou éthyle et/ou R⁹ dans la formule IV représente H ou un reste méthyle.

12. Procédé suivant la revendication 10 ou 11, **caractérisé en ce que** l'ester de formule IV est le propionate de vinyle, l'acétate de vinyle ou l'acétate d'isopropényle.

13. Procédé suivant l'une des revendications 10 à 12, **caractérisé en ce que** l'enzyme utilisé est une lipase, avantageusement une lipase extraite de *Candida rugosa, Candida cylindracea* ou *Pseudomonas cepacia.*

14. Procédé suivant l'une des revendications 10 à 13, **caractérisé en ce qu'**on utilise comme solvant un solvant organique, avantageusement le toluène.

15. Procédé suivant l'une des revendications 1 à 14, **caractérisé en ce que** les mélanges ester/alcool, lorsque la transformation enzymatique est terminée, sont séparés par une extraction sélective à l'égard du pH

16. Procédé suivant l'une des revendications 1 à 15, **caractérisé en ce que** R¹ est égal à R¹⁰, R¹⁰ étant choisi entre
H, F, Cl, Br, I, CF₃, NO₂, NH₂ ; alkyle en C₁ à C₄ ou alcényle en C₂ à C₄, ramifié ou linéaire, substitué une ou plusieurs fois ou non substitué ; OR¹¹, C(O)OR¹¹ ou SR¹¹, R¹¹ étant choisi entre
H ; alkyle en C₁ à C₄, ramifié ou linéaire, substitué une ou plusieurs fois ou non substitué ; avantageusement H, CF₃ ou CH₃,
ou bien S(O₂)NR¹²R¹³, où R¹² et R¹³ sont choisis indépendamment l'un de l'autre entre
H ; alkyle en C₁ à C₄, ramifié ou linéaire, substitué une ou plusieurs fois ou non substitué ;
R¹ étant choisi de façon particulièrement appréciée entre
H, F, Cl, OH, CH₃, C₂H₅, C₂H₃, CF₃, SCH₃, OCF₃, OCH₃, OC₂H₅, C(O)OCH₃, C(O)OC₂H₅, avantageusement m-OCH₃.

17. Procédé suivant l'une des revendications 1 à 16, **caractérisé en ce que** R² est égal à R¹⁰, R¹⁰ étant choisi entre
H, F, Cl, Br, I, SCH₃ ; alkyle en C₁ à C₄, alcényle en C₂ à C₄ ramifié ou linéaire, substitué une ou plusieurs fois ou non substitué, avantageusement CF₃ ; OR¹¹, R¹¹ étant choisi entre des restes alkyle en C₁ à C₄ ramifiés ou linéaires, substitués une ou plusieurs fois ou non substitués, avantageusement CH₃ ;
R² représentant H de façon particulièrement appréciée.

18. Procédé suivant l'une des revendications 1 à 17, **caractérisé en ce que** X est choisi entre
H, F, Cl, OH, CF₃, O-S(O₂)-C₆H₄-pCH₃ ou OC(O)R¹² avec R¹² = H ; alkyle en C₁ à C₄ ou alcényle en C₂ à C₄, ramifié ou linéaire, substitué une ou plusieurs fois ou non substitué,
avantageusement H, F, Cl, O-S(O₂)-C₆H₄-pCH₃, OC(O)R¹², R¹² étant un reste alkyle en C₁ à C₄, notamment le reste CH₃ ;
X représentant OH, F ou Cl de façon particulièrement appréciée, notamment OH.

19. Procédé suivant l'une des revendications 1 à 18, **caractérisé en ce que** R³ et R⁴ sont choisis indépendamment l'un de l'autre entre
des restes alkyle en C₁ à C₄, ramifiés ou linéaires, substitués une ou plusieurs fois ou non substitués, avantageusement le reste CH₃,
ou bien
R³ et R⁴ forment ensemble un reste cycloalkyle en C₃ à C₇, saturé ou non saturé, substitué une ou plusieurs fois ou non substitué,
R³ et R⁴ représentant chacun très avantageusement un reste CH₃.

20. Composé de formule II, 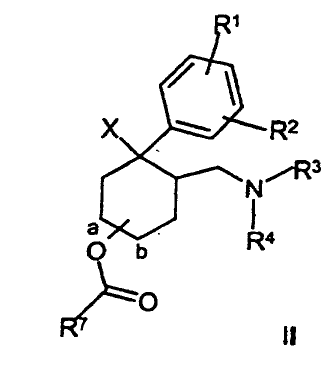 dans laquelle
X est choisi entre
H, F, Cl, Br, I, CF₃, O-S(O₂)-C₆H₄-pCH₃, OR¹⁴ ou OC(O)R¹⁴, R¹⁴ étant choisi entre
H ; alkyle en C₁ à C₁₀, alcényle en C₂ à C₁₀ ou alcynyle en C₂ à C₁₀, chacun ramifié ou linéaire, substitué une ou plusieurs fois ou non substitué ; cycloalkyle en C₃ à C₇, saturé ou non saturé, substitué une ou plusieurs fois ou non substitué, ou un hétérocycle correspondant dans lequel un atome de carbone du noyau est remplacé par N, S ou O ; alkylaryle ou alkylhétéroaryle, saturé ou non saturé, substitué une ou plusieurs fois ou non substitué ; aryle ou hétéroaryle, chacun substitué une ou plusieurs fois ou non substitué ;
R³, R⁴ sont choisis indépendamment l'un de l'autre entre
H, alkyle en C₁ à C₁₀, alcényle en C₂ à C₁₀ ou alcynyle en C₂ à C₁₀, chacun ramifié ou linéaire, substitué une ou plusieurs fois ou non substitué ; cycloalkyle en C₃ à C₇, saturé ou non saturé, substitué une ou plusieurs fois ou non substitué, ou un hétérocycle correspondant dans lequel un atome de carbone du noyau est remplacé par N, S ou O ; alkylaryle ou alkylhétéroaryle, saturé ou non saturé, substitué une ou plusieurs fois ou non substitué ; aryle ou hétéroaryle, chacun substitué une ou plusieurs fois ou non substitué ; ou bien
R³ et R⁴ forment ensemble un reste cycloalkyle en C₃ à C₇, saturé ou non saturé, substitué une ou plusieurs fois ou non substitué, ou un hétérocycle correspondant dans lequel un atome de carbone du noyau est remplacé par S, O ou NR¹⁵, R¹⁵ étant choisi entre
H, alkyle en C₁ à C₁₀, alcényle en C₂ à C₁₀ ou alcynyle en C₂ à C₁₀, chacun ramifié ou linéaire, substitué une ou plusieurs fois ou non substitué ;
R¹ et R² sont choisis indépendamment l'un de l'autre entre R¹⁰ et YR¹⁰ avec Y = alkyle en C₁ à C₁₀, alcényle en C₂ à C₁₀ ou alcynyle en C₂ à C₁₀, ramifié ou linéaire, substitué une ou plusieurs fois ou non substitué, R¹⁰ étant choisi entre
H, F, Cl, Br, I, CN, NO₂, alkyle en C₁ à C₈, alcényle en C₂ à C₈ ou alcynyle en C₂ à C₈, chacun ramifié ou linéaire, substitué une ou plusieurs fois ou non substitué ; cycloalkyle en C₃ à C₇, saturé ou non saturé, substitué une ou plusieurs fois ou non substitué, ou un hétérocycle correspondant dans lequel un atome de carbone du noyau est remplacé par S, O ou N ; aryle ou hétéroaryle, chacun substitué une ou plusieurs fois ou non substitué ;
OR¹¹, OC(O)R¹¹, OC(O)OR¹¹, OC(S)R¹¹, C(O)R¹¹, C(O)OR¹¹, C(S)R¹¹, C(S)OR¹¹, SR¹¹, S(O)R¹¹ et S(O₂)R¹¹, R¹¹ étant choisi entre
H, alkyle en C₁ à C₁₈, alcényle en C₂ à C₁₈ ou alcynyle en C₂ à C₁₈, chacun ramifié ou linéaire, substitué une ou plusieurs fois ou non substitué ; cycloalkyle en C₃ à C₇, saturé ou non saturé, substitué une ou plusieurs fois ou non substitué ; ou un hétérocycle correspondant, dans lequel un atome de carbone du noyau est remplacé par S, O ou N ; alkylaryle ou alkylhétéroaryle, saturé ou non saturé, substitué une ou plusieurs fois ou non substitué ; aryle ou hétéroaryle, chacun substitué une ou plusieurs fois ou non substitué ; ou bien
NR¹²R¹³, C(O)NR¹²R¹³ ou S(O₂)NR¹²R¹³, R¹² et R¹³ étant choisis indépendamment l'un de l'autre entre
H, alkyle en C₁ à C₁₈, alcényle en C₂ à C₁₈ ou alcynyle en C₂ à C₁₈, chacun ramifié ou linéaire, substitué une ou plusieurs fois ou non substitué, cycloalkyle en C₃ à C₇, saturé ou non saturé, substitué une ou plusieurs fois ou non substitué, ou un hétérocycle correspondant, dans lequel un atome de carbone du noyau est remplacé par S, O ou N ; alkylaryle ou alkylhétéroaryle, saturé ou non saturé, substitué une ou plusieurs fois ou non substitué ; aryle ou hétéroaryle, chacun étant substitué une ou plusieurs fois ou non substitué ;
ou bien
R¹² et R¹³ forment ensemble un reste cycloalkyle en C₃ à C₇ saturé ou non saturé, substitué une ou plusieurs fois ou non substitué, ou un hétérocycle correspondant dans lequel un atome de carbone du noyau est remplacé par S, O ou N ;
ou bien
R¹ et R² forment ensemble un groupe -CH=CH-CH=CH-, le système naphtyle formé pouvant être substitué une ou plusieurs fois,
le substituant OC(O)R⁷ est lié en position a ou b avec le noyau cyclohexane selon la formule II
et
R⁷ est choisi entre des restes alkyle en C₁ à C₆, non substitués ou substitués une ou plusieurs fois ; à l'état de base libre ou sous forme de son sel.

21. Composé suivant la revendication 20, **caractérisé en ce que** R¹ est égal à R¹⁰, R¹⁰ étant choisi entre
H, F, Cl, Br, I, CF₃, NO₂, NH₂ ; alkyle en C₁ à C₄ ou alcényle en C₂ à C₄, ramifié ou linéaire, substitué une ou plusieurs fois ou non substitué ; OR¹¹, C(O)R¹¹ ou SR¹¹, R¹¹ étant choisi entre
H ; alkyle en C₁ à C₄, ramifié ou linéaire, substitué une ou plusieurs fois ou non substitué ; avantageusement H, CF₃ ou CH₃,
ou bien S(O₂)NR¹²R¹³, où R¹² et R¹³ sont choisis indépendamment l'un de l'autre entre
H ; alkyle en C₁ à C₄, ramifié ou non ramifié, substitué une ou plusieurs fois ou non substitué ;
R¹ étant choisi de façon particulièrement appréciée entre
H, F, Cl, OH, CH₃, C₂H₅, C₂H₃, CF₃, SCH₃, OCF₃, OCH₃, OC₂H₅, C(O)OCH₃, C(O)OC₂H₅ et représentant avantageusement m-OCH₃.

22. Composé suivant la revendication 20 ou 21, **caractérisé en ce que** R² est égal à R¹⁰, R¹⁰ étant choisi entre
H, F, Cl, Br, I, SCH₃ ; alkyle en C₁ à C₄, alcényle en C₂ à C₄ ramifié ou linéaire, substitué une ou plusieurs fois ou non substitué, avantageusement CF₃ ; OR¹¹, R¹¹ étant choisi entre des restes alkyle en C₁ à C₄ ramifiés ou linéaires, substitués une ou plusieurs fois ou non substitués, avantageusement le reste CH₃ ;
R² représentant H de façon particulièrement appréciée.

23. Composé suivant l'une des revendications 20 à 22, **caractérisé en ce que** X est choisi entre
H, F, Cl, OH, CF₃, O-S(O₂)-C₆H₄-pCH₃ et OC(O)R¹² avec R¹² = H ; alkyle en C₁ à C₄ ou alcényle en C₂ à C₄, ramifié ou linéaire, substitué une ou plusieurs fois ou non substitué,
avantageusement H, F, Cl, OH, O-S(O₂)-C₆H₄-pCH₃, OC(O)R¹², avec R¹² = alkyle en C₁ à C₄, avantageusement CH₃ ;
X représentant très avantageusement OH, F ou Cl, notamment OH.

24. Composé suivant l'une des revendications 20 à 23, **caractérisé en ce que** R³ et R⁴ sont choisis indépendamment l'un de l'autre entre
des restes alkyle en C₁ à C₄, ramifiés ou linéaires, substitués une ou plusieurs fois ou non substitués, avantageusement le reste CH₃,
ou bien
R³ et R⁴ forment ensemble un reste cycloalkyle en C₃ à C₇, saturé ou non saturé, substitué une ou plusieurs fois ou non substitué,
R³ et R⁴ représentant chacun très avantageusement un reste CH₃.

25. Composé suivant l'une des revendications 20 à 24, **caractérisé en ce qu'**il est l'ester (1SR,3RS,4RS)-3-diméthylaminométhyl-4-hydroxy-4-(3-méthoxyphényl)-cyclo-hexylique d'acide butyrique, en particulier le chlorhydrate.

26. Procédé de production d'un composé suivant l'une des revendications 20 à 25, dans lequel des composés racémiques répondant à la formule I 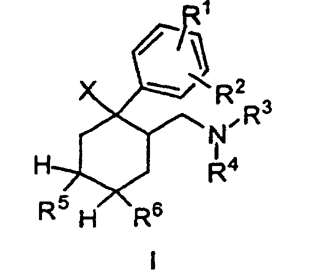 sont transformés en alcoolates avec des bases, avantageusement le tertiobutylate de potassium ou l'hydrure de sodium, dans un solvant, avantageusement le tétrahydrofuranne ou le diméthylformamide, puis les alcoolates sont transformés avec addition des halogénures d'acides correspondants en les esters racémiques de formule II, 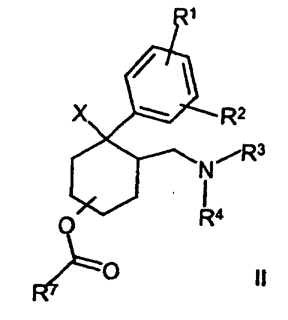 dans lesquels le substituant OC(O)R⁷ correspond à la position de R⁵ ou R⁶ dans la formule I.

27. Médicament contenant un composé suivant l'une des revendications 20 à 25 sous forme de ses diastéréoisomères et de ses énantiomères ainsi que de sa base libre ou d'un sel formé avec un acide acceptable du point de vue physiologique, en particulier le chlorhydrate.

28. Utilisation d'un composé suivant l'une des revendications 20 à 25, sous forme de ses diastéréoisomères ou de ses énantiomères ainsi que de sa base libre ou d'un sel formé avec un acide acceptable du point de vue physiologique, en particulier sous forme du chlorhydrate, pour la préparation d'un médicament destiné au traitement de la douleur, en particulier de la migraine, d'une douleur aiguë ainsi que d'une douleur neuropathique ou chronique, de réactions inflammatoires et allergiques, de dépressions, d'abus de drogues et/ou d'alcools, de la gastrite, de maladies cardiovasculaires, de maladies des voies respiratoires, de la toux, de troubles psychiques et/ou de l'épilepsie ainsi que, notamment, de l'incontinence urinaire, du prurit et/ou de la diarrhée.
